(19) Europäisches Patentamt
European Patent Office
Office européen des brevets

(11) Veröffentlichungsnummer: **0 024 560**
**B1**

(12) **EUROPÄISCHE PATENTSCHRIFT**

(45) Veröffentlichungstag der Patentschrift: **13.04.83**

(51) Int. Cl.³: **C 07 D 313/08,** A 61 K 31/335

(21) Anmeldenummer: **80104467.8**

(22) Anmeldetag: **29.07.80**

(54) **Neue 3-Amino-1-benzoxepin-Derivate und ihre Salze; Verfahren zu deren Herstellung und diese Verbindungen enthaltende Arzneimittel.**

(30) Priorität: **02.08.79 DE 2931399**

(43) Veröffentlichungstag der Anmeldung: **11.03.81 Patentblatt 81/10**

(45) Bekanntmachung des Hinweises auf die Patenterteilung: **13.04.83 Patentblatt 83/15**

(84) Benannte Vertragsstaaten: **AT BE CH DE FR GB IT LI LU NL SE**

(56) Entgegenhaltungen: **DE-A-1 593 760**

(73) Patentinhaber: **Kali-Chemie Pharma GmbH, Hans-Böckler-Allee 20 Postfach 220, D-3000 Hannover 1 (DE)**

(72) Erfinder: **Ohlendorf, Heinrich-Wilhelm Dipl.-Chem.Dr.rer.nat, Lilienstrasse 19, D-3000 Hannover 1 (DE)**
Erfinder: **Wolf, Klaus-Ullrich, Dr., Imkersweg 6, D-3165 Hänigsen (DE)**
Erfinder: **Kaupmann, Wilhelm, Dipl.-Chem. Dr. Ing., St. Ingbert-Weg 9, D-3000 Hannover-Kirchrode (DE)**
Erfinder: **Heinemann, Henning, Dipl.-Chem. Dr. rer. nat., Bergiusstrasse 18, D-3000 Hannover 51 (DE)**

(74) Vertreter: **Lauer, Dieter, Dr. et al, c/o Kali-Chemie Aktiengesellschaft Postfach 220, D-3000 Hannover 1 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 024 560 B1

BUNDESDRUCKEREI BERLIN

Neue 3-Amino-1-benzoxepin-Derivate und ihre Salze; Verfahren zu deren Herstellung und diese Verbindungen enthaltende Arzneimittel

Die Erfindung betrifft neue 3-Amino-1-benzoxepin-Derivate und ihre Salze, Verfahren zu deren Herstellung sowie diese Substanzen enthaltende pharmazeutische Zusammensetzungen.

Der vorliegenden Erfindung liegt die Aufgabe zugrunde, neue Verbindungen mit wertvollen pharmakologischen und therapeutischen Eigenschaften herzustellen.

Überraschenderweise wurde gefunden, daß die neuen 3-Amino-1-benzoxepin-Derivate eine gute Wirkung auf die Motilität des Magens haben.

Gegenstand der Erfindung sind daher neue 3-Amino-1-benzoxepin-Derivate der Formel I

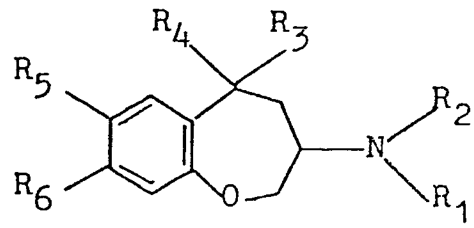

worin $R_1$ und $R_2$ unabhängig voneinander jeweils ein Wasserstoffatom, eine $C_1-C_5$-Alkylgruppe, welche gegebenenfalls einen unsubstituierten Phenylrest oder einen durch ein oder zwei Halogenatome, Methyl- oder Methoxygruppen oder eine 3,4-Methylendioxy- oder 3,4-Äthylendioxygruppe substituierten Phenylrest endständig tragen kann, eine gegebenenfalls endständig mit einer Hydroxy- oder Methoxygruppe substituierte $C_2-C_5$-Alkylgruppe oder eine $C_3-C_4$-Alkenylgruppe bedeuten, oder einer der beiden Reste $R_1$ und $R_2$ ein Wasserstoffatom oder eine $C_1-C_5$-Alkylgruppe und der andere eine $C_2-C_5$-Alkylgruppe, endständig substituiert mit einer $NR_7R_8$-Gruppe — in welcher $R_7$ und $R_8$ unabhängig voneinander jeweils ein Wasserstoffatom oder eine $C_1-C_5$-Alkylgruppe sein können oder in welcher $R_7$ und $R_8$ direkt oder über ein Heteroatom O, S oder N zu einem 5- bis 7-Ring miteinander verbunden sein können — bedeuten oder die Alkylgruppen $R_1$ und $R_2$ direkt oder über ein Heteroatom O, S oder $NR_9$ zu einem 5- bis 7-Ring miteinander verbunden sein können, wobei $R_9$ ein Wasserstoffatom, eine Methyl-, Benzyl- oder Phenylgruppe ist, einer der beiden Reste $R_3$ und $R_4$ ein Wasserstoffatom und der andere eine Hydroxygruppe ist oder beide Reste $R_3$ und $R_4$ zusammen Sauerstoff sind, $R_5$ und $R_6$ unabhängig voneinander jeweils Wasserstoffatom, Halogenatom, Alkyl-, Alkoxy- oder Alkylthiogruppe bedeuten, wobei der Alkylrest jeweils 1 bis 4 Kohlenstoffatome haben kann, oder einer der beiden Reste $R_5$ und $R_6$ Trifluormethyl- oder Nitrogruppe und der andere Wasserstoffatom sind, deren Stereoisomeren sowie die Säureadditionssalze davon.

Die 3-Amino-1-benzoxepin-Derivate der Formel I, in welchen einer der beiden Reste $R_3$ und $R_4$ ein Wasserstoffatom und der andere eine Hydroxygruppe ist, lassen sich in Racemate mit cis- bzw. trans-Konfiguration des Carbinol- und Aminrestes trennen.

Die Verbindungen der Formel I enthalten mindestens ein chirales Kohlenstoffatom und liegen in der D- und L-Form vor; die beschriebene Erfindung umfaßt auch die racemischen Gemische und die reinen D- und L-Formen der Verbindungen.

Als gegebenenfalls substituierte Alkylreste bzw. Alkenylreste $R_1$ und $R_2$ kommen gerade oder verzweigte Gruppen mit bis zu 5 Kohlenstoffatomen in Frage, so beispielsweise die Methyl-, Äthyl-, Propyl-, Isopropyl-, Butyl-, Isobutyl-, tert.-Butyl-, Pentyl-, Isopentyl-, Neopentyl-, Allyl-, 2-Butenyl- oder 3-Butenylreste. Bevorzugt sind Verbindungen, in welchen nur einer der Reste $R_1$ und $R_2$ einen substituierten Alkylrest bedeutet und der andere ein Wasserstoffatom oder Alkylrest ist.

Für $R_7$ und $R_8$ kommen die obengenannten Alkylgruppen mit 1 bis 5 Kohlenstoffatomen in Frage.

Wenn die Alkylgruppen an den Stickstoffatomen entweder direkt oder über ein Heteroatom miteinander verbunden sind, kommen z. B. in Frage Pyrrolidin, Piperidin, Azacycloheptan, Morpholin, Thiomorpholin, Piperazin und Homopiperazin, wobei letztere gegebenenfalls am Stickstoffatom mit Methyl, Benzyl oder Phenyl substituiert sein können. Bevorzugt werden die Verbindungen mit 5- bzw. 6-Ring.

Für die Substituenten $R_5$ und $R_6$ am Phenylring kommen als Halogenatome Fluor-, Chlor-, Brom oder Jodatome, insbesondere Fluor-, Chlor- oder Bromatome in Frage. Die $C_1$- bis $C_4$-Alkylreste in den Alkyl-, Alkoxy- oder Alkylthiogruppen können gerade oder verzweigt sein, wobei insbesondere bei Mehrfachsubstitution am Phenylrest der Methylrest, so Methyl, Methoxy, oder Methylthio, vorherrscht. Für die Nitro- oder Trifluormethylgruppe ist die Monosubstitution bevorzugt.

Die Verbindungen der Formel I können gewünschtenfalls durch Umsetzung mit anorganischen oder organischen Säuren in an sich bekannter Weise in ihre physiologisch verträglichen Säureadditionssalze überführt werden. Als geeignete Säuren haben sich beispielsweise Chlorwasserstoffsäure, Bromwasserstoffsäure, Schwefelsäure, Salpetersäure, Orthophosphorsäure, Maleinsäure, Cyclohexylaminosulfonsäure, Amidosulfonsäure oder p-Toluolsulfonsäure erwiesen.

Gegenstand der Erfindung ist ferner ein Verfahren zur Herstellung der 3-Amino-1-benzoxepin-Derivate der Formel I

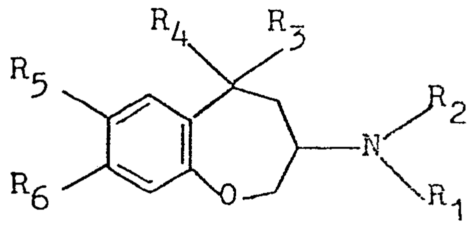

worin $R_1$, $R_2$, $R_3$, $R_4$, $R_5$ und $R_6$ die obige Bedeutung haben, deren Stereoisomeren sowie der Säureadditionssalze, dadurch gekennzeichnet, daß man 3-Amino-1-benzoxepin-5(2H)-on-Derivate der Formel II

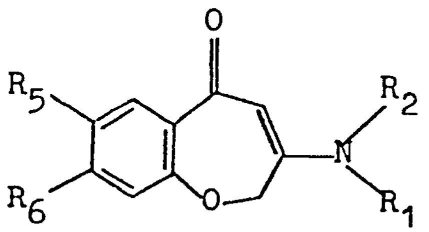

worin $R_1$, $R_2$, $R_5$ und $R_6$ die obige Bedeutung haben, in einem inerten Lösungsmittel im Temperaturbereich zwischen −70°C und der Rückflußtemperatur des eingesetzten Lösungsmittels und gegebenenfalls in Gegenwart einer anorganischen oder organischen Säure mit einem Hydridreduktionsmittel reduziert oder in einem inerten Lösungsmittel der katalytischen Hydrierung mit Wasserstoff unterwirft,

a) gewünschtenfalls die 3,4-Doppelbindung hydriert und die 5-Ketogruppe zur 5-Hydroxygruppe reduziert und die 2,3,4,5-Tetrahydro-3-amino-1-benzoxepin-5-ol-Derivate der Formel I isoliert,
b) gewünschtenfalls die 3,4-Doppelbindung hydriert und die 3-Amino-3,4-dihydro-1-benzoxepin-5(2H)-on-Derivate der Formel I isoliert,
c) gewünschtenfalls die 3-Amino-3,4-dihydro-1-benzoxepin-5(2H)-on-Derivate der Formel I zu den 2,3,4,5-Tetrahydro-3-amino-1-benzoxepin-5-ol-Derivaten der Formel I reduziert und isoliert,
d) gewünschtenfalls die unter a) bis c) erhaltenen freien Basen in die entsprechenden Säureadditionssalze überführt oder die Säureadditionssalze in die freien Basen umwandelt,
e) gewünschtenfalls die unter a) oder c) erhaltenen Verbindungen der Formel I in die rac.-cis- und rac.-trans-2,3,4,5-Tetrahydro-3-amino-1-benzoxepin-5-ol-Derivate der Formel I auftrennt und gegebenenfalls dieselben in Form ihrer Säureadditionssalze isoliert,
f) gewünschtenfalls die racemischen Verbindungen der Formel I in die optischen Antipoden spaltet,
g) gewünschtenfalls durch nachträgliche Alkylierung die N-Alkylverbindungen herstellt.

Die als Ausgangsverbindungen eingesetzten 3-Amino-1-benzoxepin-5(2H)-on-Derivate der Formel II sind in den EP-A-0 025 109 beschrieben. Sie stellen selbst pharmakologisch wertvolle Verbindungen dar, welche vor allem eine hemmende und regulierende Wirkung bei Spasmen der glatten Muskulatur im Magen-Darmkanal aufweisen.

Die Verbindungen können z. B. dadurch erhalten werden, daß man 2,3,4,5-Tetrahydro-1-benzoxepin-3,5-dion-Derivate der Formel III

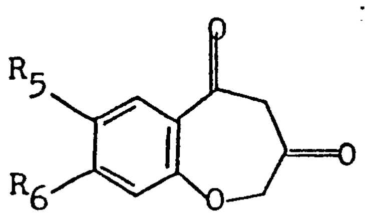

worin $R_5$ und $R_6$ die obige Bedeutung haben,

a) mit einem Amin der Formel IV

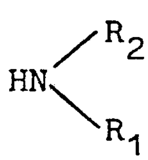

worin $R_1$ und $R_2$ die obige Bedeutung haben, in einem inerten Lösungsmittel umsetzt oder

b) mit einem Säurehalogenid in einem inerten Lösungsmittel in Verbindungen der Formel V

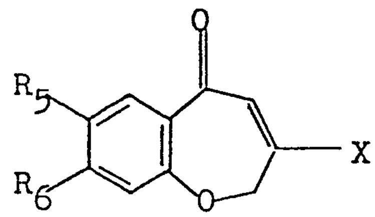

worin $R_5$ und $R_6$ die obige Bedeutung haben und X Chlor oder Brom ist, überführt und diese durch Umsetzung mit einem Amin der Formel IV in die Verbindungen der Formel II überführt, die freie Base isoliert und sie gegebenenfalls in ein Säureadditionssalz überführt oder aus deren Säureadditionssalz die freie Base isoliert.

In einer Stufe gelingt die Hydrierung der 3,4-Doppelbindung und die Reduktion der 5-Ketogruppe zur 5-Hydroxygruppe, wenn man die Verbindungen der Formel II entweder im neutralen oder schwach sauren Bereich, insbesondere im pH-Bereich 4 bis 7, mit Natriumborhydrid oder im sauren Bereich, vorzugsweise zwischen pH 3 bis 4, mit Natriumcyanoborhydrid reduziert. Als Lösungsmittel können z. B. Dioxan, Tetrahydrofuran, Dimethylformamid, Äthylenglykoldimethyläther oder Diäthylenglykoldimethyläther eingesetzt werden; man kann auch niedermolekulare Alkohole, wie Methanol, Äthanol und Isopropanol, verwenden. Zur Einstellung des jeweils günstigen pH-Bereichs können anorganische oder organische Säuren, wie z. B. Essigsäure, p-Toluolsulfonsäure, Benzolsulfonsäure, Salzsäure oder Schwefelsäure, dienen.

Die gleichzeitige Hydrierung und Reduktion gelingt auch, wenn man die Verbindungen der Formel II mittels Wasserstoff in Gegenwart von Raney-Nickel in einem protischen Lösungsmittel, vorzugsweise Äthanol oder Isopropanol, in an sich bekannter Weise reduziert.

Hierbei fallen die rac.-2,3,4,5-Tetrahydro-3-amino-1-benzoxepin-5-ole der Formel I an. Sie können in Form ihrer freien Basen oder ihrer Säureadditionssalze aus dem Reaktionsgemisch isoliert werden.

Zur Trennung in die Racemate mit cis- bzw. trans-Konfiguration des Carbinol- und Aminrestes kann man in an sich bekannter Weise diese freien Basen oder deren Säureadditionssalze in geeigneten Lösungsmitteln, wie beispielsweise niederen Alkoholen, der fraktionierten Kristallisation unterwerfen. Als besonders geeignete Säuren für die Salzbildung haben sich u. a. Maleinsäure, p-Toluolsulfonsäure und die Cyclohexylaminosulfonsäure erwiesen. Ebenso kann man diese Auftrennung durch Chromatographie der freien Basen mit geeigneten Lösungsmitteln an Kieselsäuregel oder Aluminiumoxid erreichen.

Die rac.-cis- und die rac.-trans-2,3,4,5-Tetrahydro-3-amino-1-benzoxepin-5-ole der Formel I können in an sich bekannter Weise durch Umsetzung mit geeigneten optisch aktiven Säuren, wie beispielsweise Weinsäure, o,o'-Dibenzoylweinsäure, Mandelsäure, Di-O-isopropyliden-2-oxo-L-gulonsäure und anschließende fraktionierte Kristallisation der gewonnenen Salze in ihre optisch aktiven Antipoden aufgetrennt werden. Aus diesen Salzen kann man die freien Basen freisetzen und diese gewünschtenfalls in die pharmakologisch verträglichen Salze überführen. Durch Umkristallisation aus Lösungsmitteln, wie niederen Alkoholen und/oder Äther, können die racemischen Verbindungen und ihre Isomeren sowie deren Säureadditionssalze gereinigt werden.

Die Hydrierung der 3,4-Doppelbindung in den Verbindungen der Formel II entsprechend Stufe b) gelingt im schwach sauren Bereich, vorzugsweise bei pH 4 bis 6, mittels Natriumcyanoborhydrid in Lösungsmitteln, wie z. B. niederen Alkoholen, Dioxan, Tetrahydrofuran, Dimethylformamid, Äthylenglykoldimethyläther oder Diäthylenglykoldimethyläther. Als geeignete Säuren haben sich z. B. Essigsäure, p-Toluolsulfonsäure, Benzolsulfonsäure, Salzsäure oder Schwefelsäure erwiesen. Hydrierung mit Wasserstoff in Gegenwart von Raney-Nickel in einem aprotischen Lösungsmittel, wie Toluol oder Benzol, führt ebenfalls zur selektiven Hydrierung der 3,4-Doppelbindung der Verbindung der Formel II. Durch Umsetzung mit Säuren lassen sich die Säureadditionssalze der rac.-3-Amino-3,4-dihydro-1-benzoxepin-5(2H)-on-Derivate aus den organischen Lösungsmitteln gewinnen. Die Auftrennung in die optischen Antipoden ist auf die oben beschriebene Weise möglich.

Diese Verbindungen der Formel I, welche, wie die nachfolgenden pharmakologischen Teste zeigen, selbst wertvolle pharmakologische Wirkung zeigen, sind zudem Zwischenprodukte zur Herstellung der entsprechenden 5-Hydroxyverbindungen der Formel I. Die Reduktion der Ketogruppe kann mit üblichen Reduktionsmitteln, wie z. B. im neutralen pH-Bereich mit Natriumborhydrid, Lithiumborhydrid, Lithiumaluminiumhydrid, Natrium-bis-(2-methoxy-äthoxy)-aluminiumhydrid oder Lithium-tri-sec.-butylhydrid, oder im stark sauren pH-Bereich mit Natriumcyanoborhydrid in den obengenannten Lösungsmitteln und unter Verwendung der obigen Säuren zur Einstellung des pH-Wertes durchgeführt werden. Durch Wahl geeigneter Lösungsmittel und Reduktionsmittel kann dabei eine Anreicherung des gewünschten Racemats erzielt werden.

Weiter ist es möglich, die Verbindungen der Formel I, in welchen $R_1$ und $R_2$ sowie $R_7$ und $R_8$ die Bedeutung Wasserstoffatom haben, durch nachträgliche Alkylierung in die gewünschten N-Alkylverbindungen überzuführen. Übliche Methoden sind z. B. die Umsetzung dieser Verbindungen mit Halogenalkylen(s. Houben—Weyl, Bd. XI/1 (1957), S. 24 ff.), mit Dialkylsulfat, beispielsweise Dimethyl- oder Diäthylsulfat oder Äthylendisulfat (s. a. a. O. S. 207), mit Sulfonsäureestern der Formel $R'-SO_3R$, in welcher R' beispielsweise Methyl-, Phenyl- oder 4-Methylphenyl und R eine Alkylgruppe ist (s. a. a. O. S. 217) oder mit Äthylen- und Propylenoxid (s. a. a. O. S. 311). Auch die Alkylierung mit einem Aldehyd oder Keton in Gegenwart von Natriumcyanoborhydrid als Reduktionsmittel ist durchführbar (s. J. Org. Chem. 37, 1673 (1972)).

Die neuen Verbindungen und ihre Salze weisen, soweit sie nicht wichtige Zwischenprodukte sind, wertvolle therapeutische Eigenschaften auf, vor allem zeigen sie eine ausgeprägte Wirkung bei Motilitätsstörungen im Magen-Darm-Kanal.

Es ist bekannt, daß einem großen Teil der gastroenterologischen Beschwerden funktionelle Störungen zugrunde liegen. In zunehmendem Maße werden Motilitätsstörungen, insbesondere des Magens und seiner Sphincteren, als Ursache verschiedener gastro-intestinaler Erkrankungen erkannt (s. Leber, Magen, Darm, 8 (1978), Nr. 4, S. 177—182 und 184—190 bzw. Internist 20 (1979), S. 10—17). Vor allem eine Pylorusinkompetenz, die für den duodenogastrischen Reflux verantwortlich gemacht wird, nimmt breiten Raum in der Diskussion um die pathophysiologischen Ursachen verschiedener Störungen ein (s. Dig. Diseases, 21 (1976), Nr. 2, S. 165—173). Demnach werden die Refluxgastritis, das Ulcus ventriculi und Ulcus duodeni sowie Völlegefühl, Übelkeit und epigastrische Schmerzen ohne anatomisch faßbare Ursache durch Störungen der Magenpassage hervorgerufen oder in ihrem Verlauf kompliziert.

Ziel einer hier ansetzenden Therapie ist daher die Wiederherstellung der physiologischen Magenmotilität und der ungestörten Magenpassage.

Überraschenderweise wurde gefunden, daß die erfindungsgemäßen Substanzen einen solchen Effekt besitzen. Im Tierexperiment werden unter ihrem Einfluß die peristaltischen Wellen des Magens verstärkt, wobei die Frequenz der Bewegungen zugunsten kräftiger, tiefdurchschnürender Wellen abnimmt. Aus diesen Wirkungen ergibt sich eine Verbesserung der Magenentleerung.

## Beschreibung der pharmakologischen Untersuchungsmethoden

### 1. Akute Toxizität

Die akute 7-Tage-Toxizität wird nach einmaliger Applikation intraperitoneal an der weißen nüchternen NMRI-Maus bestimmt. Die Berechnung der $LD_{50}$-Werte erfolgt über EDV durch eine Probitanalyse (L. Cavalli-Sforza, Gustav-Fischer-Verlag, Stuttgart (1964), Grundbegriffe der Biometrie, S. 153 ff.).

### 2. Prüfung der Magenperistaltik

Zur Bestimmung der Magenperistaltik werden etwa 200 g schweren, mit Ketamino-Hydrochlorid/Xylazin narkotisierten Ratten in die Vena jugularis ein Gefäß-Katheter und in die Trachea ein Trachealkatheter eingeführt. In den Magen wird eine Magensonde eingebunden, die über einen Dreiwegehahn mit einem Statham-Druckgeber (P 23 DB) verbunden wird. Der Magen wird am Pylorus und an der Cardia durch eine Ligatur verschlossen. Der Magen wird mit 3 ml 0,9%iger wäßriger NaCl-Lösung gefüllt. Die vom Magen erzeugten Druckwellen werden kontinuierlich von einem Watanabe-Multicorder (MC 641) registriert. Zur Bestimmung der Wirkung der Testsubstanzen werden diese in physiologischer Natriumchloridlösung gelöst oder in Tylose MH50 suspendiert in einer Dosis von 20 mg/kg intraperitoneal appliziert. Verglichen werden die vor und nach Gabe der Substanzen auftretenden Amplituden und Frequenzen der Druckwellen des Magens.

Die Auswertung ergibt, daß kurz nach Applikation der erfindungsgemäßen Substanzen eine beträchtliche Amplitudensteigerung auftritt. Dieser Effekt in Verbindung mit einer unterschiedlich ausgeprägten Frequenzsenkung führt zu einer verbesserten Magenpassage.

Nach den beschriebenen Methoden wurden beispielsweise folgende Substanzen untersucht:

A) rac.-cis-2,3,4,5-Tetrahydro-3-methylamino-1-benzoxepin-5-ol
A') rac.-trans-2,3,4,5-Tetrahydro-3-methylamino-1-benzoxepin-5-ol
B) rac.-cis-2,3,4,5-Tetrahydro-3-methylamino-8-chlor-1-benzoxepin-5-ol
B') rac.-trans-2,3,4,5-Tetrahydro-3-methylamino-8-chlor-1-benzoxepin-5-ol
C) rac.-cis-2,3,4,5-Tetrahydro-3-isopropylamino-1-benzoxepin-5-ol
C') rac.-trans-2,3,4,5-Tetrahydro-3-isopropylamino-1-benzoxepin-5-ol
D) rac.-cis-2,3,4,5-Tetrahydro-3-benzylamino-1-benzoxepin-5-ol
D') rac.-trans-2,3,4,5-Tetrahydro-3-benzylamino-1-benzoxepin-5-ol

E) rac.-cis-2,3,4,5-Tetrahydro-3-(γ-dimethylamino-propylamino)-1-benzoxepin-5-ol
E') rac.-trans-2,3,4,5-Tetrahydro-3-(γ-dimethylaminopropylamino)-1-benzoxepin-5-ol
F) rac.-cis-2,3,4,5-Tetrahydro-3-diäthylamino-1-benzoxepin-5-ol
F') rac.-trans-2,3,4,5-Tetrahydro-3-diäthylamino-1-benzoxepin-5-ol
G) rac.-cis-2,3,4,5-Tetrahydro-3-methylamino-7-chlor-1-benzoxepin-5-ol
G') rac.-trans-2,3,4,5-Tetrahydro-3-methylamino-7-chlor-1-benzoxepin-5-ol
H) rac.-cis-2,3,4,5-Tetrahydro-3-(n-butylamino)-1-benzoxepin-5-ol
H') rac.-trans-2,3,4,5-Tetrahydro-3-(n-butylamino)-1-benzoxepin-5-ol
I) rac.-cis-2,3,4,5-Tetrahydro-3-morpholino-1-benzoxepin-5-ol
I') rac.-trans-2,3,4,5-Tetrahydro-3-morpholino-1-benzoxepin-5-ol
K) rac.-cis-2,3,4,5-Tetrahydro-3-pyrrolidino-1-benzoxepin-5-ol
K') rac.-trans-2,3,4,5-Tetrahydro-3-pyrrolidino-1-benzoxepin-5-ol
L) rac.-cis-2,3,4,5-Tetrahydro-3-phenäthylamino-1-benzoxepin-5-ol
L') rac-trans-2,3,4,5-Tetrahydro-3-phenäthylamino-1-benzoxepin-5-ol
M) rac.-cis-2,3,4,5-Tetrahydro-3-methylamino-7,8-dichlor-1-benzoxepin-5-ol
M') rac.-trans-2,3,4,5-Tetrahydro-3-methylamino-7,8-dichlor-1-benzoxepin-5-ol
N) rac.-cis-2,3,4,5-Tetrahydro-3-amino-1-benzoxepin-5-ol
N') rac.-trans-2,3,4,5-Tetrahydro-3-amino-1-benzoxepin-5-ol
O) rac.-cis-2,3,4,5-Tetrahydro-3-dimethylamino-1-benzoxepin-5-ol
O') rac.-trans-2,3,4,5-Tetrahydro-3-dimethylamino-1-benzoxepin-5-ol
1) rac.-3-Methylamino-3,4-dihydro-1-benzoxepin-5(2H)-on
2) rac.-3-Isopropylamino-3,4-dihydro-1-benzoxepin-5(2H)-on
3) rac.-3-Benzylamino-3,4-dihydro-1-benzoxepin-5(2H)-on
4) rac.-3-(γ-Dimethylamino-propylamino)-3,4-dihydro-1-benzoxepin-5(2H)-on
5) rac.-3-Dimethylamino-3,4-dihydro-1-benzoxepin-5(2H)-on
6) rac.-3-(γ-Dimethylamino-propylamino)-7-chlor-3,4-dihydro-1-benzoxepin-5(2H)-on
7) rac.-3-(n-Butylamino)-3,4-dihydro-1-benzoxepin-5(2H)-on

Tabelle

Magendruckmessung

| Substanz | Amplituden-steigerung um Faktor | Frequenz-senkung in % | $LD_{50}$ i. p. (mg/kg) |
|---|---|---|---|
| A | 5,6 | 3,0 | 342 |
| A' | 8,8 | 12,7 | 285 |
| B | 9,6 | 31,0 | 200 |
| B' | 4,5 | 4,0 | 83 |
| C | 6,3 | 13,0 | 272 |
| C' | 12,5 | 23,0 | 664 |
| D | 7,6 | 21,0 | 92 |
| D' | 12,4 | 31,0 | 113 |
| E | 22,9 | 32,0 | 664 |
| E' | 25,6 | 18,0 | 590 |
| F | 9,5 | 13,0 | 183 |
| F' | 8,9 | 28,0 | 219 |
| G | 13,5 | 10,0 | n. b. |
| G' | 11,0 | 26,0 | n. b. |
| H | 32,3 | 36,0 | n. b. |

Fortsetzung

| Substanz | Amplitudensteigerung um Faktor | Frequenzsenkung in % | $LD_{50}$ i. p. (mg/kg) |
|---|---|---|---|
| H' | 15,3 | 8,0 | n. b. |
| I | 0 | 0 | n. b. |
| I' | 2,2 | 12,0 | n. b. |
| K | 9,7 | 10,0 | n. b. |
| K' | 18,3 | 20,0 | n. b. |
| L | 12,6 | 21,0 | n. b. |
| L' | 15,9 | 10,0 | n. b. |
| M | 6,9 | 22,0 | n. b. |
| M' | 6,0 | 9,0 | n. b. |
| N | 8,1 | 0 | n. b. |
| N' | 12,0 | 30,0 | n. b. |
| O | 5,8 | 7,0 | n. b. |
| O' | 17,2 | 8,0 | n. b. |
| 1 | 1,6 | 8,0 | 137 |
| 2 | 2,6 | 16,0 | 183 |
| 3 | 3,6 | 7,0 | 253 |
| 4 | 3,7 | 25,0 | 272 |
| 5 | 1,3 | 12,0 | 285 |
| 6 | 3,4 | 29,0 | 296 |
| 7 | 6,6 | 28,0 | n. b. |

Die vorhergehende Tabelle enthält die gemessenen Werte. Aus ihnen geht eindeutig hervor, daß schon geringe Dosen der erfindungsgemäßen Substanzen und deren Säureadditionssalze eine signifikante Verstärkung der peristaltischen Wellen des Magens bewirken, wobei die hohe Wirksamkeit und die geringe Toxizität der Substanzen auf eine gute Verträglichkeit derselben hinweisen. Ein weiterer Vorteil ist der beobachtete rasche Wirkungseintritt.

Die pharmakologisch beobachteten Wirkungen lassen darauf schließen, daß die erfindungsgemäßen Substanzen am Menschen Störungen der Magen-Darmfunktion, wie beispielsweise Pylorusstenosen, duodenogastrischen Reflux sowie atonische Zustände, beheben werden. Ein günstiger therapeutischer Effekt ist ferner bei verschiedenen funktionellen Beschwerden zu erwarten, die zu Oberbauchschmerzen, Übelkeit, Völlegefühl und sonstigen Mißempfindungen führen. Hierzu gehören die Symptome bei Ulcus ventriculi und Ulcus duodeni, bei Gastritis und nervösem Reizmagen. Ebenso wird die in der Röntgendiagnostik des Magen-Darm-Kanals erwünschte gesteigerte Magenpassage der Kontrastmittel erreicht.

Die Arzneimittelzubereitungen enthalten die Substanzen der Formel I oder deren Pharmakologisch verträgliche Salze als Wirkstoff in Kombination mit üblichen pharmakologisch verträglichen Trägerstoffen und/oder Verdünnungsmitteln. Die Arzneimittel können oral oder parenteral verabreicht werden und liegen als Tabletten, Kapseln, Sirups, Trockenpulver, injizierbare und infundierbare

Lösungen oder Suspensionen vor. Sie können aber auch als Suppositorien konfektioniert werden. Im allgemeinen werden oral verabreichbare Präparate bevorzugt.

Die Dosierung der erfindungsgemäßen Arzneipräparate hängt von verschiedenen Faktoren ab, wie von der Art und der Schwere der Krankheit oder von der verwendeten Verbindung. Im allgemeinen genügt bei oraler Gabe eine Einzeldosis von 0,1—20, insbesondere 0,5—10 mg, um zufriedenstellende Ergebnisse zu erhalten.

## Beispiel I

Kapseln mit 10 mg rac.-cis-2,3,4,5-Tetrahydro-3-methylamino-1-benzoxepin-5-ol als Wirkstoff

Zusammensetzung:

| | |
|---|---|
| Wirkstoff | 10 Teile |
| Lactose | 65 Teile |
| Maisstärke, getrocknet | 40 Teile |
| lösliche Stärke | 4 Teile |
| Magnesiumstearat | 1 Teil |
| | 120 Teile |

Herstellungsvorschrift:

Der Wirkstoff wird mit Lactose und Maisstärke vermischt. Die entstandene Mischung wird mit einer 15%igen wäßrigen Lösung der löslichen Stärke durchfeuchtet und granuliert. Die feuchte Masse wird durch ein 1,6-mm-Sieb passiert, bei 40°C auf Horden getrocknet und anschließend durch ein 1,0-mm-Sieb passiert. Nach dem Vermischen des Granulats mit Magnesiumstearat wird die entstandene Mischung in Mengen von 120 mg verkapselt, so daß jede Kapsel 10 mg Wirkstoff enthält.

## Beispiel 1

Zu einer im Eisbad gekühlten Lösung von 18,8 g (0,1 Mol) 3-Methylamino-1-benzoxepin-5(2H)-on in einem Gemisch aus 125 ml Dioxan und 125 ml Essigsäure wird unter Kühlung Natriumborhydrid in kleinen Portionen bis zur Beendigung der Reaktion zugefügt (etwa 0,4 Mol). Die Reaktionslösung wird in Eiswasser gegossen, mit Natriumcarbonat alkalisch gestellt und mit Dichlormethan extrahiert. Die organische Lösung wird mit gesättigter Kochsalzlösung gewaschen und über Natriumsulfat getrocknet. Nach Abziehen des Lösungsmittels erhält man 16,4 g (85% d. Th.) rac.-2,3,4,5-Tetrahydro-3-methylamino-1-benzoxepin-5-ol als öligen Rückstand.

Der Fp. des Hydrochlorids beträgt 152—157° C (Isopropanol).

Zur Trennung der Isomeren wird das oben erhaltene Öl in Isopropanol aufgenommen und mit überschüssiger Maleinsäure versetzt. Durch fraktionierte Krstallisation der Maleinate aus Isopropanol und anschließende Überführung in die Hydrochloride erhält man rac.-cis-2,3,4,5-Tetrahydro-3-methylamino-1-benzoxepin-5-ol-hydrochlorid. Fp. 190° C (Methanol/Äther) und rac.-trans-2,3,4,5-Tetrahydro-3-methylamino-1-benzoxepin-5-ol-hydrochlorid. Fp. 170—173° C (Methanol/Äther).

## Beispiel 2

18,8 g (0,1 Mol) 3-Methylamino-1-benzoxepin-5(2H)-on werden mit 40 g Raney-Nickel in 400 ml Äthanol 5 Stunden bei einem Wasserstoffdruck von 55 bar gerührt. Anschließend wird der Katalysator abfiltriert, das Lösungsmittel abgedampft, und man erhält 7,7 g (40% d. Th.) rac.-2,3,4,5-Tetrahydro-3-methylamino-1-benzoxepin-5-ol als öligen Rückstand. Die Verbindungen können wie vorher beschrieben getrennt und charakterisiert werden.

## Beispiel 3

Zu einer Lösung von 94,2 g (0,5 Mol) 3-Methylamino-1-benzoxepin-5(2H)-on in einem Gemisch aus 300 ml Dioxan und 300 ml Eisessig werden unter Kühlung 18,9 g (0,3 Mol) Natriumcyanoborhydrid in kleinen Portionen so zugefügt, daß die Temperatur nicht über 25°C steigt. Anschließend wird bis zur Beendigung der Reaktion bei Raumtemperatur gerührt und dann die Reaktionslösung in Eiswasser gegossen, mit Natriumcarbonat alkalisch gestellt und mit Dichlormethan extrahiert. Die organische

Phase wird mit gesättigter Kochsalzlösung gewaschen und über Natriumsulfat getrocknet. Durch Einleiten von Chlorwasserstoff wird das Hydrochlorid des rac.-3-Methylamino-3,4-dihydro-1-benzoxepin-5(2H)-on ausgefällt und anschließend abgesaugt. Die Ausbeute beträgt 89,9 g (79% d. Th.), Fp. 184—186° C (Methanol/Äther).

## Beispiel 4

Zu einer Lösung von 2,3 g (0,01 Mol) 3-(n-Butylamino)-1-benzoxepin-5(2H)-on in 10 ml Methanol fügt man eine Spur Bromkresolgrün. Dazu wird eine Lösung von Chlorwasserstoff in Methanol (etwa 3 n) getropft bis der Indikator umschlägt. Anschließend wird die Lösung von 0,65 g Natriumcyanoborhydrid (0,01 Mol) in 10 ml Methanol langsam bei Raumtemperatur zugegeben und noch 1—2 h bis zur Beendigung der Reaktion gerührt. Dabei wird durch tropfenweise Zugabe einer Lösung von Chlorwasserstoff in Methanol die gelbe Indikatorfarbe aufrechterhalten. Die Lösung wird eingedampft, mit Wasser aufgenommen, durch Einleiten von Dimethylamin alkalisch gestellt und mit Äther extrahiert. Die ätherischen Lösungen werden vereinigt und mit Natriumsulfat getrocknet. Durch Einleiten von Chlorwasserstoff wird das rac.-3-(n-Butylamino)-3,4-dihydro-1-benzoxepin-5(2H)-on-hydrochlorid ausgefällt. Das Produkt wird abgesaugt und umkristallisiert. Die Ausbeute beträgt 2,0 g (74,1% d. Th.), Fp. 154—158° C (Isopropanol/Äther).

## Beispiel 5

27,9 g (0,1 Mol) 3-Phenäthylamino-1-benzoxepin-5(2H)-on werden mit 25 g Raney-Nickel in 350 ml Toluol 20 Stunden bei einem Wasserstoffdruck von 150 bar gerührt. Anschließend wird der Katalysator abfiltriert, das Lösungsmittel abgedampft und der Rückstand mit Methanol aufgenommen. Nach Einleiten von Chlorwasserstoff werden 14,6 g (46% d. Th.) rac.-3-Phenäthylamino-3,4-dihydro-1-benzoxepin-5(2H)-on-hydrochlorid mit Fp. 160° C (Zers.) aus Methanol erhalten.

## Beispiel 6

Auf die in den Beispielen 3—5 beschriebene Weise kann man

3-Methylamino-7-methyl-1-benzoxepin-5(2H)-on
3-Methylamino-7-äthyl-1-benzoxepin-5(2H)-on
3-Methylamino-7-brom-1-benzoxepin-5(2H)-on
3-Methylamino-7-methoxy-1-benzoxepin-5(2H)-on
3-Methylamino-8-methoxy-1-benzoxepin-5(2H)-on
3-Pyrrolidino-1-benzoxepin-5(2H)-on
3-Methylamino-7,8-dichlor-1-benzoxepin-5(2H)-on
3-Methylamino-7,8-dimethyl-1-benzoxepin-5(2H)-on
3-Isopropylamino-1-benzoxepin-5(2H)-on
3-Benzylamino-1-benzoxepin-5(2H)-on
3-Dimethylamino-1-benzoxepin-5(2H)-on
3-Piperidino-1-benzoxepin-5(2H)-on
3-Morpholino-1-benzoxepin-5(2H)-on
3-(γ-Dimethylamino-propylamino)-7-chlor-1-benzoxepin-5(2H)-on
3-(γ-Dimethylamino-propylamino)-1-benzoxepin-5(2H)-on
3-(β-Dimethylamino-äthylamino)-1-benzoxepin-5(2H)-on
3-Amino-1-benzoxepin-5(2H)-on
3-(β-Methoxy-äthylamino)-1-benzoxepin-5(2H)-on

in folgende Verbindungen überführen:

| | Fp. °C |
|---|---|
| rac.-3-Methylamino-3,4-dihydro-7-methyl-1-benzoxepin-5(2H)-on-hydrochlorid | 183—185*) |
| rac.-3-Methylamino-3,4-dihydro-7-äthyl-1-benzoxepin-5(2H)-on-hydrochlorid | 146—148 |
| rac.-3-Methylamino-3,4-dihydro-7-brom-1-benzoxepin-5(2H)-on-hydrochlorid | 193—195*) |
| rac.-3-Methylamino-3,4-dihydro-7-methoxy-1-benzoxepin-5(2H)-on-hydrochlorid | 178—180*) |
| rac.-3-Methylamino-3,4-dihydro-8-methoxy-1-benzoxepin-5(2H)-on-hydrochlorid | 168—170 |

| | Fp. °C |
|---|---|
| rac.-3-Pyrrolidino-3,4-dihydro-1-benzoxepin-5(2H)-on-hydrochlorid | 128—130 |
| rac.-3-Methylamino-3,4-dihydro-7,8-dichlor-1-benzoxepin-5(2H)-on-hydrochlorid | 194*) |
| rac.-3-Methylamino-3,4-dihydro-7,8-dimethyl-1-benzoxepin-5(2H)-on-hydrochlorid | 206*) |
| rac.-3-Isopropylamino-3,4-dihydro-1-benzoxepin-5(2H)-on-hydrochlorid | 171—175 |
| rac.-3-Benzylamino-3,4-dihydro-1-benzoxepin-5(2H)-on-hydrochlorid | 141—145 |
| rac.-3-Dimethylamino-3,4-dihydro-1-benzoxepin-5(2H)-on-maleinat | 107—109 |
| rac.-3-Piperidino-3,4-dihydro-1-benzoxepin-5(2H)-on-hydrochlorid | 154—156 |
| rac.-3-Morpholino-3,4-dihydro-1-benzoxepin-5(2H)-on-hydrochlorid | 148—150 |
| rac.-3-(γ-Dimethylamino-propylamino)-3,4-dihydro-7-chlor-1-benzoxepin-5(2H)-on-dihydrochlorid-monohydrat | 150—154 |
| rac.-3-(γ-Dimethylamino-propylamino)-3,4-dihydro-1-benzoxepin-5(2H)-on-dihydrochlorid | 154—158 |
| rac.-3-(β-Dimethylamino-äthylamino)-3,4-dihydro-1-benzoxepin-5(2H)-on-dihydrochlorid | 150—157 |
| rac.-3-Amino-3,4-dihydro-1-benzoxepin-5(2H)-on-hydrochlorid | 211*) |
| rac.-3-(β-Methoxy-äthylamino)-3,4-dihydro-1-benzoxepin-5(2H)-on-hydrochlorid | 133—135 |

*) Zersetzung

## Beispiel 7

Zu einer Lösung von 33,5 g (0,1 Mol) rac.-3-(γ-Dimethylamino-propylamino)-3,4-dihydro-1-benzoxepin-5(2H)-on-dihydrochlorid in 300 ml Methanol wird unter Kühlung Natriumborhydrid bis zur Beendigung der Reaktion in kleinen Portionen zugegeben. Die Reaktionslösung wird mit Salzsäure angesäuert und zur Trockne gedampft. Der Rückstand wird mit Wasser aufgenommen, mit wäßriger Ammoniaklösung alkalisch gestellt und mit Dichlormethan extrahiert. Nach Waschen der organischen Phase mit gesättigter Kochsalzlösung, Trocknen über Natriumsulfat und Eindampfen erhält man 23,8 g (90% d. Th.) rac.-2,3,4,5-Tetrahydro-3-(γ-Dimethylamino-propylamino)-1-benzoxepin-5-ol als öligen Rückstand.

Durch Chromatographie an Kieselgel und anschließende Bildung der Dimaleinate erhält man rac.-cis-2,3,4,5-Tetrahydro-3-(γ-dimethylamino-propylamino)-1-benzoxepin-5-ol-dimaleinat mit Fp. 169—170°C (Methanol) und rac.-trans-2,3,4-5-Tetrahydro-3-(γ-dimethylamino-propylamino)-1-benzoxepin-5-ol-dimaleinat mit Fp. 157—159°C (Methanol).

## Beispiel 8

Zu einer siedenden Lösung von 80 g (0,5 Mol) rac.-3-Methylamino-3,4-dihydro-1-benzoxepin-5(2H)-on in einem Gemisch aus 550 ml Toluol und 300 ml Hexan werden 550 ml einer 1molaren Lösung von Lithium-tri-sec.-butylborhydrid in Tetrahydrofuran so zugetropft, daß die Lösung am Sieden gehalten wird. Nach beendetem Zutropfen wird eine weitere Stunde zum Sieden erhitzt. Nach dem Abkühlen wird durch Zutropfen methanolischer Salzsäure sauer gestellt, anschließend die methanolische Phase abgetrennt und das Toluol/Hexan-Gemisch nochmals mit methanolischer Salzsäure extrahiert. Die Extrakte werden eingedampft, mit Dichlormethan und Natriumcarbonatlösung (10%) aufgenommen, die organische Phase mit gesättigter Kochsalzlösung gewaschen, über Natriumsulfat getrocknet und zur Trockne gedampft. Man erhält 67,6 g (70% d. Th.) rac.-2,3,4,5-Tetrahydro-3-methylamino-1-benzoxepin-5-ol als öligen Rückstand, in dem das rac.-cis-2,3,4,5-Tetrahydro-3-methylamino-1-benzoxepin-5-ol mit 82 Teilen zu 18 Teilen rac.-trans-2,3,4,5-Tetrahydro-3-methylamino-1-benzoxepin-5-ol überwiegt. Die Verbindungen können wie vorher beschrieben getrennt und charakterisiert werden.

## Beispiel 9

Zu einer Lösung von 3,9 g (0,02 Mol) rac.-trans-2,3,4,5-Tetrahydro-3-methylamino-1-benzoxepin-5-ol in 80 ml Acetonitril und 17,1 ml wäßriger Formaldehydlösung (35%) werden 3,9 g (0,06 Mol) Natriumcyanoborhydrid gegeben. Anschließend werden 2,1 ml Eisessig zugefügt und 2 h bei Raumtemperatur gerührt. Die Lösung wird mit 270 ml Äther verdünnt, mit verdünnter Natronlauge gewaschen, über Natriumsulfat getrocknet und eingedampft. Der Rückstand wird mit Methanol aufgenommen und das rac.-trans-2,3,4,5-Tetrahydro-3-dimethylamino-1-benzoxepin-5-ol als Maleinat isoliert. Die Ausbeute beträgt 3,9 g (60% d. Th.), Fp. 157—158°C (Methanol/Äther).

Beispiel 10

Auf den in den vorhergehenden Beispielen beschriebene Weise kann man aus

3-Amino-1-benzoxepin-5(2H)-on
3-(n-Butylamino)-1-benzoxepin-5(2H)-on
3-Benzylamino-1-benzoxepin-5(2H)-on
3-Phenäthylamino-1-benzoxepin-5(2H)-on
3-Dimethylamino-1-benzoxepin-5(2H)-on
3-Diäthylamino-1-benzoxepin-5(2H)-on
3-Pyrrolidino-1-benzoxepin-5(2H)-on
3-Piperidino-1-benzoxepin-5(2H)-on
3-Morpholino-1-benzoxepin-5(2H)-on
3-Methylamino-7-äthyl-1-benzoxepin-5(2H)-on
3-Methylamino-7-chlor-1-benzoxepin-5(2H)-on
3-Methylamino-8-chlor-1-benzoxepin-5(2H)-on
3-Methylamino-7-brom-1-benzoxepin-5(2H)-on
3-Methylamino-7-methoxy-1-benzoxepin-5(2H)-on
3-Methylamino-7-chlor-8-methyl-1-benzoxepin-5(2H)-on
3-(γ-Dimethylamino-propylamino)-7-chlor-1-benzoxepin-5(2H)-on
3-(β,β-Dimethyl-γ-dimethylamino-propylamino)-1-benzoxepin-5(2H)-on
3-(β-Methoxy-äthylamino)-1-benzoxepin-5(2H)-on
3-Methylamino-7-methyl-1-benzoxepin-5(2H)-on
3-Methylamino-8-methoxy-1-benzoxepin-5(2H)-on
3-Methylamino-7,8-dichlor-1-benzoxepin-5(2H)-on
3-Methylamino-7,8-dimethyl-1-benzoxepin-5(2H)-on

folgende Verbindungen herstellen:

| | Fp. °C |
|---|---|
| rac.-cis-2,3,4,5-Tetrahydro-3-amino-1-benzoxepin-5-ol-hydrochlorid | 222*) |
| rac.-trans-2,3,4,5-Tetrahydro-3-amino-1-benzoxepin-5-ol-hydrochlorid | 160—162 |
| rac.-cis-2,3,4,5-Tetrahydro-3-(n-butylamino)-1-benzoxepin-5-ol-toluol-4-sulfonat | 148—150 |
| rac.-trans-2,3,4,5-Tetrahydro-3-(n-butylamino)-1-benzoxepin-5-ol-toluol-4-sulfonat | 187—189 |
| rac.-cis-2,3,4,5-Tetrahydro-3-benzylamino-1-benzoxepin-5-ol-maleinat | 176—178 |
| rac.-trans-2,3,4,5-Tetrahydro-3-benzylamino-1-benzoxepin-5-ol-maleinat | 133—135 |
| rac.-cis-2,3,4,5-Tetrahydro-3-phenäthylamino-1-benzoxepin-5-ol-maleinat | 162—164 |
| rac.-trans-2,3,4,5-Tetrahydro-3-phenäthylamino-1-benzoxepin-5-ol-maleinat | 182—184 |
| rac.-cis-2,3,4,5-Tetrahydro-3-dimethylamino-1-benzoxepin-5-ol-maleinat | 176—179 |
| rac.-trans-2,3,4,5-Tetrahydro-3-dimethylamino-1-benzoxepin-5-ol-maleinat | 157—158 |
| rac.-cis-2,3,4,5-Tetrahydro-3-diäthylamino-1-benzoxepin-5-ol-toluol-4-sulfonat | 185—187 |
| rac.-trans-2,3,4,5-Tetrahydro-3-diäthylamino-1-benzoxepin-5-ol-toluol-4-sulfonat | 130—131 |
| rac.-cis-2,3,4,5-Tetrahydro-3-pyrrolidino-1-benzoxepin-5-ol-toluol-4-sulfonat | 163—165 |
| rac.-trans-2,3,4,5-Tetrahydro-3-pyrrolidino-1-benzoxepin-5-ol-toluol-4-sulfonat | 158—160 |
| rac.-cis-2,3,4,5-Tetrahydro-3-piperidino-1-benzoxepin-5-ol-toluol-4-sulfonat | 178—179 |
| rac.-trans-2,3,4,5-Tetrahydro-3-piperidino-1-benzoxepin-5-ol-toluol-4-sulfonat | 142—144 |
| rac.-cis-2,3,4,5-Tetrahydro-3-morpholino-1-benzoxepin-5-ol-toluol-4-sulfonat | 154—156 |
| rac.-trans-2,3,4,5-Tetrahydro-3-morpholino-1-benzoxepin-5-ol-toluol-4-sulfonat | 145—146 |
| rac.-cis-2,3,4,5-Tetrahydro-3-methylamino-7-äthyl-1-benzoxepin- | |

| | Fp. °C |
|---|---|
| 5-ol-hydrochlorid | 138—140 |
| rac.-trans-2,3,4,5-Tetrahydro-3-methylamino-7-äthyl-1-benzoxepin-5-ol-hydrochlorid | 197—198 |
| rac.-cis-2,3,4,5-Tetrahydro-3-methylamino-7-chlor-1-benzoxepin-5-ol-hydrochlorid | 172—173 |
| rac.-trans-2,3,4,5-Tetrahydro-3-methylamino-7-chlor-1-benzoxepin-5-ol-hydrochlorid | 242*) |
| rac.-cis-2,3,4,5-Tetrahydro-3-methylamino-8-chlor-1-benzoxepin-5-ol-hydrochlorid | 194—195 |
| rac.-trans-2,3,4,5-Tetrahydro-3-methylamino-8-chlor-1-benzoxepin-5-ol-hydrochlorid | 189—190 |
| rac.-cis-2,3,4,5-Tetrahydro-3-methylamino-7-brom-1-benzoxepin-5-ol-hydrochlorid | 186—188 |
| rac.-trans-2,3,4,5-Tetrahydro-3-methylamino-7-brom-1-benzoxepin-5-ol-hydrochlorid | 243*) |
| rac.-cis-2,3,4,5-Tetrahydro-3-methylamino-7-methoxy-1-benzoxepin-5-ol-hydrochlorid | 198—200 |
| rac.-trans-2,3,4,5-Tetrahydro-3-methylamino-7-methoxy-1-benzoxepin-5-ol-hydrochlorid | 223—225 |
| rac.-cis-2,3,4,5-Tetrahydro-3-methylamino-7-chlor-8-methyl-1-benzoxepin-5-ol-hydrochlorid | 233*) |
| rac.-trans-2,3,4,5-Tetrahydro-3-methylamino-7-chlor-8-methyl-1-benzoxepin-5-ol-hydrochlorid | 243*) |
| rac.-cis-2,3,4,5-Tetrahydro-3-($\gamma$-dimethylamino-propylamino)-7-chlor-1-benzoxepin-5-ol-dihydrochlorid | 168—170 |
| rac.-cis-2,3,4,5-Tetrahydro-3-($\beta$,$\beta$-dimethyl-$\gamma$-dimethylamino-propylamino)-benzoxepin-5-ol-dimaleinat | 151—153 |
| rac.-trans-2,3,4,5-Tetrahydro-3-($\beta$,$\beta$-dimethyl-$\gamma$-dimethylamino-propylamino)-1-benzoxepin-5-ol-dimaleinat | 122—124 |
| rac.-cis-2,3,4,5-Tetrahydro-3-($\beta$-methoxyäthylamino)-1-benzoxepin-5-ol-cyclohexylaminosulfonat | 162—164 |
| rac.trans-2,3,4,5-Tetrahydro-3-($\beta$-methoxyäthylamino)-1-benzoxepin-5-ol-cyclohexylaminosulfonat | 132—133 |
| rac.-cis-2,3,4,5-Tetrahydro-3-methylamino-7-methyl-1-benzoxepin-5-ol-hydrochlorid | 199—200 |
| rac.-trans-2,3,4,5-Tetrahydro-3-methylamino-7-methyl-1-benzoxepin-5-ol-hydrochlorid | 204—205 |
| rac.-cis-2,3,4,5-Tetrahydro-3-methylamino-8-methoxy-1-benzoxepin-5-ol-hydrochlorid | 152—153 |
| rac.-trans-2,3,4,5-Tetrahydro-3-methylamino-8-methoxy-1-benzoxepin-5-ol-hydrochlorid | 139—141 |
| rac.-cis-2,3,4,5-Tetrahydro-3-methylamino-7,8-dichlor-1-benzoxepin-5-ol-hydrochlorid | 245*) |
| rac.-trans-2,3,4,5-Tetrahydro-3-methylamino-7,8-dichlor-1-benzoxepin-5-ol-hydrochlorid | 246*) |
| rac.-cis-2,3,4,5-Tetrahydro-3-methylamino-7,8-dimethyl-1-benzoxepin-5-ol-hydrochlorid | 198*) |
| rac.-trans-2,3,4,5-Tetrahydro-3-methylamino-7,8-dimethyl-1-benzoxepin-5-ol-hydrochlorid | 208*) |

*) Zersetzung

**Patentansprüche für die Vertragsstaaten: BE, CH, DE, FR, GB, IT, LI, LU, NL, SE**

1. Neue 3-Amino-1-benzoxepin-Derivate der Formel I

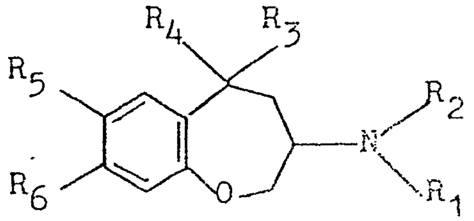

worin $R_1$ und $R_2$ unabhängig voneinander jeweils ein Wasserstoffatom, eine $C_1-C_5$-Alkylgruppe, welche gegebenenfalls einen unsubstituierten Phenylrest oder einen durch ein oder zwei Halogenatome, Methyl- oder Methoxygruppen oder eine 3,4-Methylendioxy- oder 3,4-Äthylendioxygruppe substituierten Phenylrest endständig tragen kann, eine gegebenenfalls endständig mit einer Hydroxy- oder Methoxygruppe substituierte $C_2-C_5$-Alkylgruppe oder eine $C_3-C_4$-Alkenylgruppe bedeuten, oder einer der beiden Reste $R_1$ und $R_2$ ein Wasserstoffatom oder eine $C_1-C_5$-Alkylgruppe und der andere eine $C_2-C_5$-Alkylgruppe, endständig substituiert mit einer $NR_7R_8$-Gruppe — in welcher $R_7$ und $R_8$ unabhängig voneinander jeweils ein Wasserstoffatom oder eine $C_1-C_5$-Alkylgruppe sein können oder in welcher $R_7$ und $R_8$ direkt oder über ein Heteroatom O, S oder N zu einem 5- bis 7-Ring miteinander verbunden sein können — bedeuten oder die Alkylgruppen $R_1$ und $R_2$ direkt oder über ein Heteroatom O, S oder $NR_9$ zu einem 5- bis 7-Ring miteinander verbunden sein können, wobei $R_9$ ein Wasserstoffatom, eine Methyl-, Benzyl- oder Phenylgruppe ist, einer der beiden Reste $R_3$ und $R_4$ ein Wasserstoffatom und der andere eine Hydroxygruppe ist oder beide Reste $R_3$ und $R_4$ zusammen Sauerstoff sind, $R_5$ und $R_6$ unabhängig voneinander jeweils Wasserstoffatom, Halogenatom, Alkyl-, Alkoxy- oder Alkylthiogruppe bedeuten, wobei der Alkylrest jeweils 1 bis 4 Kohlenstoffatome haben kann, oder einer der beiden Reste $R_5$ und $R_6$ Trifluormethyl- oder Nitrogruppe und der andere Wasserstoffatom sind, deren Stereoisomeren sowie die Säureadditionssalze davon.

2. rac.-cis- bzw. rac.-trans-2,3,4,5-Tetrahydro-3-A-1-benzoxepin-5-ole, worin A die Bedeutung Amino, Methylamino, Dimethylamino, Diäthylamino, β-Methoxyäthylamino, Isopropylamino, n-Butylamino, Benzylamino, Phenäthylamino- Morpholino, Pyrrolidino, Piperidino, γ-Dimethylamino-propylamino oder β,β-Dimethyl-γ-dimethylamino-propylamino hat.

3. rac.-cis- bzw. rac.-trans-2,3,4,5-Tetrahydro-3-methylamino-7B,8C-1-benzoxepin-5-ole, worin B Wasserstoff, Chlor, Brom, Methyl, Äthyl oder Methoxy und C Wasserstoff, Chlor, Methyl oder Methoxy bedeuten.

4. rac.-cis-2,3,4,5-Tetrahydro-3-(γ-dimethylamino-propylamino)-7-chlor-1-benzoxepin-5-ol.

5. rac.-3-A-3,4-dihydro-1-benzoxepin-5(2H)-one, worin A die Bedeutung Amino, Methylamino, Dimethylamino, Isopropylamino, n-Butylamino, Benzylamino, Phenäthylamino, Morpholino, Pyrrolidino, Piperidino, β-Methoxyäthylamino, β-Dimethylamino-äthylamino oder -γ-Dimethylamino-propylamino hat.

6. rac.-3-Methylamino-3,4-dihydro-7B,8C-1-benzoxepin-5(2H)one, worin B Wasserstoff, Chlor, Brom, Methyl, Äthyl oder Methoxy und C Wasserstoff, Chlor, Methyl oder Methoxy bedeuten.

7. rac.-(γ-Dimethylamino-propyl-amino)-3,4-dihydro-7-chlor-1-benzoxepin-5(2H)-on.

8. Verfahren zur Herstellung der 3-Amino-1-benzoxepin-Derivate der Formel I gemäß Anspruch 1, dadurch gekennzeichnet, daß man 3-Amino-1-benzoxepin-5(2H)-on-Derivate der Formel II

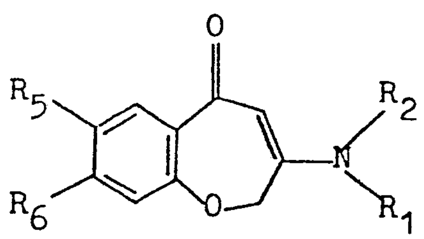

worin $R_1$, $R_2$, $R_5$ und $R_6$ die obige Bedeutung haben, in einem inerten Lösungsmittel im Temperaturbereich zwischen −70°C und der Rückflußtemperatur des eingesetzten Lösungsmittels und gegebenenfalls in Gegenwart einer anorganischen oder organischen Säure mit einem Hydridreduktionsmittel reduziert oder in einem inerten Lösungsmittel der katalytischen Hydrierung mit Wasserstoff unterwirft,

a) gewünschtenfalls die 3,4-Doppelbindung hydriert und die 5-Ketogruppe zur 5-Hydroxygruppe reduziert und die 2,3,4,5-Tetrahydro-3-amino-1-benzoxepin-5-ol-Derivate der Formel I isoliert,

b) gewünschtenfalls die 3,4-Doppelbindung hydriert und die 3-Amino-3,4-dihydro-1-benzoxepin-5(2H)-on-Derivate der Formel I isoliert,
c) gewünschtenfalls die 3-Amino-3,4-dihydro-1-benzoxepin-5(2H)-on-Derivate der Formel I zu den 2,3,4,5-Tetrahydro-3-amino-1-benzoxepin-5-ol-Derivaten der Formel I reduziert und isoliert,
d) gewünschtenfalls die unter a) bis c) erhaltenen freien Basen in die entsprechenden Säureadditionssalze überführt oder die Säureadditionssalze in die freien Basen umwandelt,
e) gewünschtenfalls die unter a) oder c) erhaltenen Verbindungen der Formel I in die rac.-cis- und rac.-trans-2,3,4,5-Tetrahydro-3-amino-1-benzoxepin-5-ol-Derivate der Formel I auftrennt und gegebenenfalls dieselben in Form ihrer Säureadditionssalze isoliert,
f) gewünschtenfalls die racemischen Verbindungen der Formel I in die optischen Antipoden spaltet,
g) gewünschtenfalls durch nachträgliche Alkylierung die N-Alkylverbindungen herstellt.

9. Verfahren nach Anspruch 8, dadurch gekennzeichnet, daß man die Hydrierung der 3,4-Doppelbindung und die Reduktion der 5-Ketogruppe gemäß Stufe a) im neutralen oder schwach sauren pH-Bereich mit Natriumborhydrid durchführt.

10. Verfahren nach Anspruch 9, dadurch gekennzeichnet, daß man die Reduktion im sauren pH-Bereich mit Natriumcyanoborhydrid durchführt.

11. Verfahren nach Anspruch 9, dadurch gekennzeichnet, daß man die Hydrierung mit Wasserstoff in Gegenwart von Raney-Nickel in einem protischen Lösungsmittel, vorzugsweise Äthanol oder Isopropanol durchführt.

12. Verfahren nach Anspruch 8, dadurch gekennzeichnet, daß man die Hydrierung der 3,4-Doppelbindung entsprechend Stufe b) im schwach sauren Bereich mittels Natriumcyanoborhydrid durchführt.

13. Verfahren nach Anspruch 12, dadurch gekennzeichnet, daß man die Hydrierung mit Wasserstoff in Gegenwart von Raney-Nickel in einem aprotischen Lösungsmittel, vorzugsweise Toluol oder Benzol durchführt.

14. Verfahren nach Anspruch 8, dadurch gekennzeichnet, daß man die Reduktion der 5-Ketogruppe zur 5-Hydroxygruppe im neutralen pH-Bereich mit Natriumborhydrid, Lithiumborhydrid, Lithiumaluminiumhydrid, Natrium-bis-(2-methoxy-äthoxy)-aluminiumhydrid oder Lithium-tri-sec.-butylborhydrid gemäß Stufe c) durchführt.

15. Verfahren nach Anspruch 14, dadurch gekennzeichnet, daß man die Reduktion im stark sauren pH-Bereich mit Natriumcyanoborhydrid durchführt.

16. Verfahren nach den Ansprüchen 8 bis 10, 12, 14 bis 15, dadurch gekennzeichnet, daß man als Lösungsmittel niedermolekulare Alkohole, Dioxan, Tetrahydrofuran, Dimethylformamid, Äthylenglykoldimethyläther oder Diäthylenglykoldimethyläther verwendet und den pH-Bereich gegebenenfalls mit Essigsäure, p-Toluolsulfonsäure, Benzolsulfonsäure, Salzsäure oder Schwefelsäure einstellt.

17. Arzneimittel, bestehend aus einer oder mehreren Verbindungen gemäß Anspruch 1 und üblichen Träger- und Hilfssubstanzen.

**Patentansprüche für den Vertragsstaat: AT**

1. Verfahren zur Herstellung der 3-Amino-1-benzoxepin-Derivate der Formel I

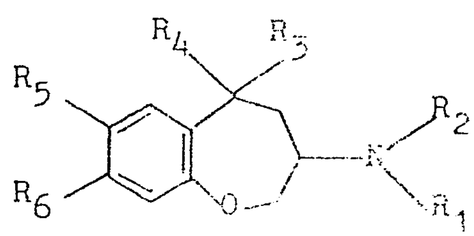

worin $R_1$ und $R_2$ unabhängig voneinander jeweils ein Wasserstoffatom, eine $C_1-C_5$-Alkylgruppe, welche gegebenenfalls einen unsubstituierten Phenylrest oder einen durch ein oder zwei Halogenatome, Methyl- oder Methoxygruppen oder eine 3,4-Methylendioxy- oder 3,4-Äthylendioxygruppe substituierten Phenylrest endständig tragen kann, eine gegebenenfalls endständig mit einer Hydroxy- oder Methoxygruppe substituierte $C_2-C_5$-Alkylgruppe oder eine $C_3-C_4$-Alkenylgruppe bedeuten, oder einer der beiden Reste $R_1$ und $R_2$ ein Wasserstoffatom oder eine $C_1-C_5$-Alkylgruppe und der andere eine $C_2-C_5$-Alkylgruppe, endständig substituiert mit einer $NR_7R_8$-Gruppe — in welcher $R_7$ und $R_8$ unabhängig voneinander jeweils ein Wasserstoffatom oder eine $C_1-C_5$-Alkylgruppe sein können oder in welcher $R_7$ und $R_8$ direkt oder über ein Heteroatom O, S oder N zu einem 5- bis 7-Ring miteinander verbunden sein können — bedeuten oder die Alkylgruppen $R_1$ und $R_2$ direkt oder über ein Heteroatom O, S oder $NR_9$ zu einem 5- bis 7-Ring miteinander verbunden sein können, wobei $R_9$ ein Wasserstoffatom, eine Methyl-, Benzyl- oder Phenylgruppe ist, einer der beiden Reste $R_3$ und $R_4$ ein

Wasserstoffatom und der andere eine Hydroxygruppe ist oder beide Reste $R_3$ und $R_4$ zusammen Sauerstoff sind, $R_5$ und $R_6$ unabhängig voneinander jeweils Wasserstoffatom, Halogenatom, Alkyl-, Alkoxy- oder Alkylthiogruppe bedueten, wobei der Alkylrest jeweils 1 bis 4 Kohlenstoffatome haben kann, oder einer der beiden Reste $R_5$ und $R_6$ Trifluormethyl- oder Nitrogruppe und der andere Wasserstoffatom sind, deren Stereoisomeren sowie die Säureadditionssalze davon, dadurch gekennzeichnet, daß man 3-Amino-1-benzoxepin-5(2H)-on-Derivate der Formel II

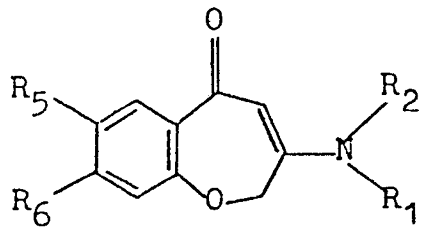

worin $R_1$, $R_2$, $R_5$ und $R_6$ die obige Bedeutung haben, in einem inerten Lösungsmittel im Temperaturbereich zwischen −70° C und der Rückflußtemperatur des eingesetzten Lösungsmittels und gegebenenfalls in Gegenwart einer anorganischen oder organischen Säure mit einem Hydridreduktionsmittel reduziert oder in einem inerten Lösungsmittel der katalytischen Hydrierung mit Wasserstoff unterwirft,

a) gewünschtenfalls die 3,4-Doppelbindung hydriert und die 5-Ketogruppe zur 5-Hydroxygruppe reduziert und die 2,3,4,5-Tetrahydro-3-amino-1-benzoxepin-5-ol-Derivate der Formel I isoliert,
b) gewünschtenfalls die 3,4-Doppelbindung hydriert und die 3-Amino-3,4-dihydro-1-benzoxepin-5(2H)-on-Derivate der Formel I isoliert,
c) gewünschtenfalls die 3-Amino-3,4-dihydro-1-benzoxepin-5(2H)-on-Derivate der Formel I zu den 2,3,4,5-Tetrahydro-3-amino-1-benzoxepin-5-ol-Derivaten der Formel I reduziert und isoliert,
d) gewünschtenfalls die unter a) bis c) erhaltenen freien Basen in die entsprechenden Säureadditionssalze überführt oder die Säureadditionssalze in die freien Basen umwandelt,
e) gewünschtenfalls die unter a) oder c) erhaltenen Verbindungen der Formel I in die rac.-cis- und rac.-trans-2,3,4,5-Tetrahydro-3-amino-1-benzoxepin-5-ol-Derivate der Formel I auftrennt und gegebenenfalls dieselben in Form ihrer Säureadditionssalze isoliert,
f) gewünschtenfalls die racemischen Verbindungen der Formel I in die optischen Antipoden spaltet,
g) gewünschtenfalls durch nachträgliche Alkylierung die N-Alkylverbindungen herstellt.

2. Verfahren nach Anspruch 1, dadurch gekennzeichnet, daß man die Hydrierung der 3,4-Doppelbindung entsprechend Stufe b) im schwach sauren Bereich mittels Natriumcyanoborhydrid durchführt.

3. Verfahren nach Anspruch 1, dadurch gekennzeichnet, daß man die Reduktion der 5-Ketogruppe zur 5-Hydroxygruppe im neutralen pH-Bereich mit Natriumborhydrid, Lithiumborhydrid, Lithiumaluminiumhydrid, Natrium-bis-(2-methoxy-äthoxy)-aluminiumhydrid oder Lithiumtri-sec.-butylborhydrid gemäß Stufe c) durchführt.

4. Verfahren nach Anspruch 1, dadurch gekennzeichnet, daß als Verbindungen der Formel I rac.-cis- bzw. rac.-trans-2,3,4,5-Tetrahydro-2-A-1-benzoxepin-5-ole hergestellt werden, worin A die Bedeutung Amino, Methylamino, Dimethylamino, Diäthylamino, $\beta$-Methoxyäthylamino, Isopropylamino, n-Butylamino, Benzylamino, Phenäthylamino, Morpholino, Pyrrolidino, Piperidino, $\gamma$-Dimethylamino-propylamino oder $\beta,\beta$-Dimethyl-$\gamma$-dimethylamino-propylamino hat.

5. Verfahren nach Anspruch 1, dadurch gekennzeichnet, daß als Verbindungen der Formel I rac.-cis- bzw. rac.-trans-2,3,4,5-Tetrahydro-3-methylamino-7B,8C-1-benzoxepin-5-ole hergestellt werden, worin B Wasserstoff, Chlor, Brom, Methyl, Äthyl oder Methoxy und C Wasserstoff, Chlor, Methyl oder Methoxy bedeuten.

6. Verfahren nach Anspruch 1, dadurch gekennzeichnet, daß als Verbindungen der Formel I rac.-3-A-3,4-dihydro-1-benzoxepin-5(2H)-one hergestellt werden, worin A die Bedeutung Amino, Methylamino, Dimethylamino, Isopropylamino, n-Butylamino, Benzylamino, Phenäthylamino, Morpholino, Pyrrolidino, Piperidino, $\beta$-Methoxyäthylamino, $\beta$-Dimethylamino-äthylamino oder $\gamma$-Dimethylamino-propylamino hat.

7. Verfahren nach Anspruch 1, dadurch gekennzeichnet, daß als Verbindungen der Formel I rac.-3-Methylamino-3,4-dihydro-7B,8C-1-benzoxepin-5(2H)-one hergestellt werden, worin B Wasserstoff, Chlor, Brom, Methyl, Äthyl oder Methoxy und C Wasserstoff, Chlor, Methyl oder Methoxy bedeuten.

**Claims for the Contracting states: BE, CH, DE, FR, GB, IT, LI, LU, NL, SE**

1. New 3-amino-1-benzoxepine derivatives of the formula I

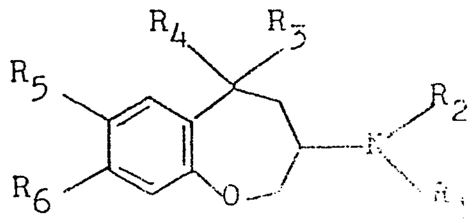

wherein $R_1$ and $R_2$ independently of one another each denote a hydrogen atom, a $C_1-C_5$-alkyl group, which can optionally be end-substituted with an unsubstituted phenyl radical or a phenyl radical which is substituted by one or two halogen atoms, methyl or methoxy groups or by a 3,4-methylenedioxy or 3,4-ethylenedioxy group, a $C_2-C_5$-alkyl group which is optionally end-substituted by a hydroxyl or methoxy group, or a $C_3-C_4$-alkenyl group, or one of the two radicals $R_1$ and $R_2$ can be a hydrogen atom or a $C_1-C_5$-alkyl group and the other a $C_2-C_5$-alkyl group which is end-substituted with an $NR_7R_8$ group, in which $R_7$ and $R_8$ independently of one another can each be a hydrogen atom or a $C_1-C_5$-alkyl group, or in which $R_7$ and $R_8$ can be linked to one another directly or via a hetero-atom O, S or N to form a 5- membered to 7-membered ring, or the alkyl groups $R_1$ and $R_2$ can be linked to one another directly or via a hetero-atom O, S or $NR_9$ to form a 5-membered to 7-membered ring, $R_9$ being a hydrogen atom or a methyl, benzyl or phenyl group, one of the two radicals $R_3$ and $R_4$ is a hydrogen atom and the other is a hydroxyl group, or both radicals $R_3$ and $R_4$ together are oxygen, $R_5$ and $R_6$ independently of one another each denote a hydrogen atom, halogen atom, alkyl, alkoxy or alkylthio group, it being possible for the alkyl radical in each case to have 1 to 4 carbon atoms, or one of the two radicals $R_5$ and $R_6$ is trifluoromethyl or nitro group and the other a hydrogen atom, their stereoisomers as well as acid addition salts thereof.

2. Racemic cis- and racemic trans-2,3,4,5-tetrahydro-3-A-1-benzoxepin-5-ols, wherein A denotes amino, methylamino, dimethylamino, diethylamino, $\beta$-methoxyethylamino, isopropylamino, n-butylamino, benzylamino, phenethylamino, morpholino, pyrrolidino, piperidino, $\gamma$-dimethylamino-propylamino, or $\beta,\beta$-dimethyl-$\gamma$-dimethylamino-propylamino.

3. Racemic cis- and racemic trans-2,3,4,5-tetrahydro-3-methylamino-7B,8C-1-benzoxepin-5-ols, wherein B denotes hydrogen, chlorine, bromine, methyl, ethyl or methoxy and C denotes hydrogen, chlorine, methyl or methoxy.

4. Racemic cis-2,3,4,5-tetrahydro-3-($\gamma$-dimethylamino-propylamino)-7-chloro-1-benzoxepin-5-ol.

5. Racemic 3-A-3,4-dihydro-1-benzoxepin-5(2H)-ones, wherein A denotes amino, methylamino, dimethylamino, isopropylamino, n-butylamino, benzylamino, phenethylamino, morpholino, pyrrolidino, piperidino, $\beta$-methoxy-ethylamino, $\beta$-dimethylamino-ethylamino or -$\gamma$-dimethylamino-propylamino.

6. Racemic 3-methylamino-3-4-dihydro-7B,8C-1-benzoxepin-5(2H)-ones, wherein B denotes hydrogen, chlorine, bromine, methyl, ethyl or methoxy and C denotes hydrogen, chlorine, methyl or methoxy.

7. Racemic ($\gamma$-dimethylamino-propyl-amino)-3,4-dihydro-7-chloro-1-benzoxepin-5(2H)-one.

8. Process for the preparation of 3-amino-1-benzoxepine derivatives of the formula I according to claim 1, characterised in that 3-amino-1-benzoxepin-5(2H)-one derivatives of the formula II

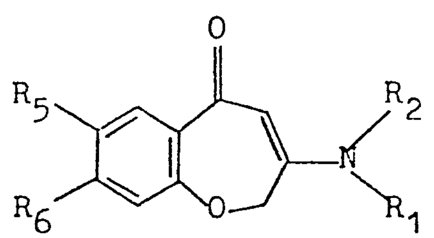

wherein $R_1$, $R_2$, $R_5$ and $R_6$ have the above meaning, are reduced with a hydride reducing agent in an inert solvent in the temperature range between $-70°C$ and the reflux temperature of the solvent employed and if appropriate in the presence of an inorganic or organic acid, or are subjected to catalytic hydrogenation with hydrogen in an inert solvent,

a) if desired, the 3,4-double bond is hydrogenated and the 5-keto group is reduced to the 5-hydroxy group and the 2,3,4,5-tetrahydro-3-amino-1-benzoxepin-5-ol derivatives of the formula I are isolated,

b) if desired, the 3,4-double bond is hydrogenated and the 3-amino-3,4-dihydro-1-benzoxepin-5(2H)-one derivatives of the formula I are isolated,
c) if desired, the 3-amino-3,4-dihydro-1-benzoxepin-5(2H)-one derivatives of the formula I are reduced to the 2,3,4,5-tetrahydro-3-amino-1-benzoxepin-5-ol derivatives of the formula I and isolated,
d) if desired, the free bases obtained under a) to c) are converted into the corresponding acid addition salts or the acid addition salts are converted into the free bases,
e) if desired, the compounds of the formula I obtained under a) or c) are separated into the racemic cis- and racemic trans-2,3,4,5-tetrahydro-3-amino-1-benzoxepin-5-ol derivatives of the formula I and these are optionally isolated in the form of their acid addition salts,
f) if desired, the racemic compounds of the formula I are resolved into the optical antipodes,
g) if desired, the N-alkyl-compounds are produced by subsequent alkylation.

9. Process according to Claim 8 characterised in that the hydrogenation of the 3,4-double bond and the reduction of the 5-keto group according to stage a) is carried out in a neutral or slightly acid pH range with sodium borohydride.

10. Process according to Claim 9, characterised in that the reduction is carried out in an acid pH range with sodium cyanoborohydride.

11. Process according to Claim 9, characterised in that the hydrogenation is carried out with hydrogen in the presence of Raney nickel in a protic solvent, preferably ethanol or isopropanol.

12. Process according to Claim 8, characterised in that the hydrogenation of the 3,4-double bond corresponding to stage b) is carried out in a weakly acid range by means of sodium cyanoborohydride.

13. Process according to Claim 12, characterised in that the hydrogenation is carried out with hydrogen in the presence of Raney nickel in an aprotic solvent, preferably toluene or benzene.

14. Process according to Claim 8, characterised in that the reduction of the 5-keto group to the 5-hydroxy group is carried out in neutral pH range with sodium borohydride, lithium borohydride, lithium aluminium hydride, sodium bis-(2-methoxy-ethoxy)-aluminium hydride or lithium tri-sec.-butylborohydride according to stage c.

15. Process according to Claim 14, characterised in that the reduction is carried out in a strongly acid pH range with sodium cyanoborohydride.

16. Process according to Claims 8 to 10, 12, 14 to 15, characterised in that the solvent used is a low-molecular alcohol, dioxane, tetrahydrofuran, dimethylformamide ethylene glycol dimethyl ether or diethylene glycol dimethyl ether and the pH range ist adjusted if necessary, with acetic acid, p-toluenesulphonic acid, benzenesulphonic acid, hydrochloric acid or sulphuric acid.

17. Medicaments consisting of one or more compounds according to Claim 1 and customary carriers and auxiliary substances.

**Claims for the Contracting state: AT**

1. Process for the preparation of 3-amino-1-benzoxepine derivatives of the formula I

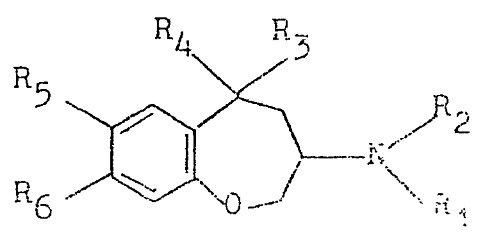

wherein $R_1$ and $R_2$ independently of one another each denote a hydrogen atom, a $C_1-C_5$-alkyl group, which can optionally be end-substituted with an unsubstituted phenyl radical or a phenyl radical which is substituted by one or two halogen atoms, methyl or methoxy groups or by a 3,4-methylenedioxy or 3,4-ethylenedioxy group, a $C_2-C_5$-alkyl group which is optionally end-substituted by a hydroxyl or methoxy group, or a $C_3-C_4$-alkenyl group, or one of the two radicals $R_1$ and $R_2$ can be an hydrogen atom or a $C_1-C_5$-alkyl group and the other a $C_2-C_5$-alkyl group which is end-substituted with an $NR_7R_8$ group, in which $R_7$ and $R_8$ independently of one another can each be a hydrogen atom or a $C_1-C_5$-alkyl group, or in which $R_7$ and $R_8$ can be linked to one another directly or via a hetero-atom O, S or N to form a 5- membered to 7-membered ring, or the alkyl groups $R_1$ and $R_2$ can be linked to one another directly or via a hetero-atom O, S or $NR_9$ to form a 5-membered to 7-membered ring, $R_9$ being a hydrogen atom or a methyl, benzyl or phenyl group, one of the two radicals $R_3$ and $R_4$ is a hydrogen atom and the other is a hydroxyl group, or both radicals $R_3$ and $R_4$ together are oxygen, $R_5$ and $R_6$ independently of one another each denote a hydrogen atom, halogen atom, alkyl, alkoxy or alkylthio group, it being possible for the alkyl radical in each case to have 1 to 4 carbon atoms, or one of the two radicals $R_5$ and $R_6$ is

trifluoromethyl or nitro group and the other is hydrogen atom, their stereoisomers as well as acid addition salts thereof, characterised in that 3-amino-1-benzoxepin-5(2H)-one derivatives of the formula II

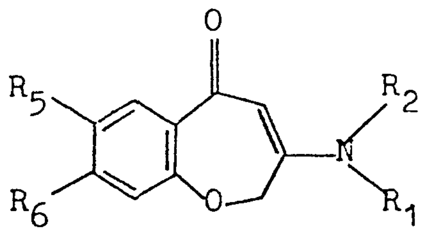

wherein $R_1$, $R_2$, $R_5$ and $R_6$ have the above meaning, are reduced with a hydride reducing agent in an inert solvent in the temperature range between −70°C and the reflux temperature of the solvent employed and if appropriate in the presence of an inorganic or organic acid, or are subjected to catalytic hydrogenation with hydrogen in an inert solvent,

a) if desired, the 3,4-double bond is hydrogenated, and the 5-keto group is reduced to the 5-hydroxy group and the 2,3,4,5-tetrahydro-3-amino-1-benzoxepin-5-ol derivatives of the formula I are isolated,
b) if desired, the 3,4-double bond is hydrogenated and the 3-amino-3,4-dihydro-1-benzoxepin-5(2H)-one derivatives of the formula I are isolated,
c) if desired, the 3-amino-3,4-dihydro-1-benzoxepin-5(2H)-one derivatives of the formula I are reduced to the 2,3,4,5-tetrahydro-3-amino-1-benzoxepin-5-ol derivatives of the formula I and isolated,
d) if desired, the free bases obtained under a) to c) are converted into the corresponding acid addition salts or the addition salts are converted into the free bases,
e) if desired, the compounds of the formula I obtained under a) or c) are separated into the racemic cis- and racemic trans-2,3,4,5-tetrahydro-3-amino-1-benzoxepin-5-ol derivatives of the formula I and these are optionally isolated in the form of their acid addition salts,
f) if desired, the racemic compounds of the formula I are resolved into the optical antipodes,
g) if desired, the N-alkyl-compounds are produced by subsequent alkylation.

2. Process according to Claim 1, characterised in that the hydrogenation of the 3,4-double bond corresponding to stage b) is carried out in a weakly acid range by means of sodium cyanoborohydride.

3. Process according to Claim 1, characterised in that the reduction of the 5-keto group to the 5-hydroxy group is carried out in a neutral pH range with sodium borohydride, lithium borohydride, lithium aluminium hydride, sodium bis-(2-methoxy-ethoxy)-aluminium hydride or lithium tri-sec.-butylborohydride according to stage c).

4. Process according to Claim 1, characterised in that there are produced as compounds of formula I, racemic cis- and racemic trans-2,3,4,5-tetrahydro-3-A-1-benzoxepin-5-ols, wherein A denotes amino, methyl-amino, dimethylamino, diethylamino, β-methoxyethylamino, isopropylamino, n-butylamino, benzylamino, phenethylamino, morpholino, pyrrolidino, piperidino, γ-dimethylamino-propylamino or β,β-dimethyl-γ-dimethylamino-propylamino.

5. Process according to Claim 1, characterised in that there are produced as compounds of formula I, racemic cis- and racemic trans-2,3,4,5-tetrahydro-3-methylamino-7B,8C-1-benzoxepin-5-ols, wherein B denotes hydrogen, chlorine, bromine, methyl, ethyl or methoxy and C denotes hydrogen, chlorine, methyl or methoxy.

6. Process according to Claim 1, characterised in that there are produced as compounds of formula I, racemic 3-A-3,4-dihydro-1-benzoxepin-5(2H)-ones, wherein A denotes amino, methylamino, dimethylamino, isopropylamino, n-butylamino, benzylamino, phenethylamino, morpholino, pyrrolidino, piperidino, β-methoxyethylamino, β-dimethylamino-ethylamino or -γ-dimethylamino-propylamino.

7. Process according to claim 1, characterised in that there are produced as compounds of formula I, racemic 3-methylamino-3,4-dihydro-7B,8C-1-benzoxepin-5(2H)-ones, wherein B denotes hydrogen, chlorine, bromine, methyl ethyl or methoxy and C denotes hydrogen, chlorine, methyl or methoxy.

**Revendications pour les Etats contractants: BE, CH, DE, FR, GB, IT, LI, LU, NL, SE**

1. Nouveaux dérivés de 3-amino-1-benzoxépines de Formule I

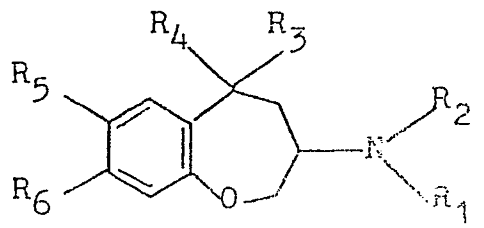

où $R_1$ et $R_2$ sont, indépendamment l'un de l'autre, chacun un atome d'hydrogène, un groupe alkyle en $C_1-C_5$, lequel peut éventuellement porter en position terminale un radical phényle non-substitué ou un radical phényle substitué par un ou deux atomes d'halogène, groupes méthyle ou groupes méthoxy, ou par un groupe 3,4-méthylènedioxy ou 3,4-éthylènedioxy, un groupe alkyle en $C_2-C_5$ éventuellement substitué en position terminale par un groupe hydroxy ou méthoxy, ou un groupe alcényle en $C_3-C_4$, ou bien l'un des deux radicaux $R_1$ et $R_2$ est un atome d'hydrogène ou un groupe alkyle en $C_1-C_5$ et l'autre est un groupe alkyle en $C_2-C_5$ substitué en position terminale par un groupe $NR_7R_8$ — dans lequel $R_7$ et $R_8$ peuvent, indépendamment l'un de l'autre, être chacun un atome d'hydrogène ou un groupe alkyle en $C_1-C_5$, ou où $R_7$ et $R_8$ peuvent être liés l'un à l'autre, directement ou par l'intermédiaire d'un hétéroatome O, S ou N pour donner un noyau pentagonal à heptagonal, ou bien les groupes alkyle $R_1$ et $R_2$ peuvent être liés l'un à l'autre directement ou par l'intermédiaire d'un hétéroatome O, S ou $NR_9$ pour donner un noyau pentagonal à heptagonal, $R_9$ étant un atome d'hydrogène, un groupe méthyle, benzyle, ou phényle, l'un des deux radicaux $R_3$ et $R_4$ étant un atome d'hydrogène et l'autre un groupe hydroxy, ou bien les deux radicaux $R_3$ et $R_4$ sont ensemble l'oxygène, $R_5$ et $R_6$ sont, indépendamment l'un de l'autre, chacun un atome d'hydrogène, un atome d'halogène, un groupe alkyle, alcoxy ou alkylthio, où le radical alkyle peut chaque fois avoir 1 à 4 atomes de carbone, ou bien l'un des deux radicaux $R_5$ et $R_6$ est le groupe trifluorométhyle ou nitro, et l'autre est un atome d'hydrogène, leurs stéréoisomères et leurs sels d'addition avec un acide.

2. rac.-cis- ou rac.-trans-2,3,4,5-tétrahydro-3-A-1-benzoxépine-5-ols où A signifie l'un des radicaux amino, méthylamino, diméthylamino, diéthylamino, β-méthoxyéthylamino, isopropylamino, n-butylamino, benzylamino, phénéthylamino, morpholino, pyrrolidino, pipéridino, γ-diméthylamino-propylamino ou β,β-diméthyl-γ-diméthylamino-propylamino.

3. rac.-cis- ou rac.-trans-2,3,4,5-tétrahydro-3-méthylamino-7B,8C-1-benzoxépine-5-ols où B est l'hydrogène, le chlore, le brome, le méthyle, l'éthyle ou le méthoxy, et C est l'hydrogène, le chlore, le méthyle ou le méthoxy.

4. rac.-cis-2,3,4,5-tétrahydro-3-(γ-diméthylamino-propylamino)-7-chloro-1-benzoxépine-5-ol.

5. rac.-3-A-3,4-dihydro-1-benzoxépine-5(2H)-ones, où A est l'un des radicaux amino, méthylamino, diméthylamino, isopropylamino, n-butylamino, benzylamino, phénéthylamino, morpholino, pyrrolidino, pipéridino, β-méthoxyéthylamino, β-diméthylamino-éthylamino, ou -γ-diméthylamino-propylamino.

6. rac.-3-méthylamino-3,4-dihydro-7B,8C-1-benzoxépine-5(2H)-ones, où B est l'hydrogène, le chlore, le brome, le méthyle, l'éthyle ou le méthoxy, et C est l'hydrogène, le chlore, le méthyle ou le méthoxy.

7. rac.-(γ-diméthylamino-propyl-amino)-3,4-dihydro-7-chloro-1-benzoxépine-5(2H)-one.

8. Procédé de préparation des dérivés de 3-amino-1-benzoxépines de Formule I selon la revendication 1, caractérisé en ce qu'il consiste à réduire avec un agent réducteur aux hydrures des dérivés de la 3-amino-1-benzoxépine-5(2H)-one de Formule II

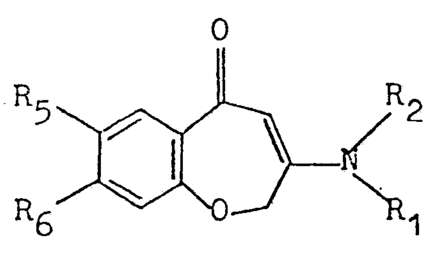

où $R_1$, $R_2$, $R_5$ et $R_6$ ont la signification ci-dessus, dans un solvant inerte dans la plage de températures comprise entre −70°C et la température de reflux du solvant utilisé et éventuellement en présence d'un acide organique ou inorganique, ou à les soumettre dans un solvant inerte à une hydrogénation catalytique avec de l'hydrogène,

a) si on le souhaite, à hydrogéner la double liaison 3,4 et à réduire le groupe 5-céto en le groupe

5-hydroxy, et à isoler les dérivés du 2,3,4,5-tétrahydro-3-amino-1-benzoxépine-5-ol de Formule I,

b) si on le souhaite, à hydrogéner la double liaison 3,4 et à isoler les dérivés de la 3-amino-3,4-dihydro-1-benzoxépine-5(2H)-one de Formule I,

c) si on le souhaite, à réduire les dérivés de la 3-amino-3,4-dihydro-1-benzoxépine-5(2H)-one de Formule I en les dérivés du 2,3,4,5-tétrahydro-3-amino-1-benzoxépine-5-ol de Formule I et à les isoler,

d) si on le souhaite, à convertir les bases libres obtenues en a) à c) en les sels d'addition avec un acide correspondants ou à convertir les sels d'addition avec un acide en les bases libres,

e) si on le souhaite, à séparer les composés de Formule I obtenus en a) ou c) en les dérivés du rac.-cis- et du rac.-trans-2,3,4,5-tétrahydro-3-amino-1-benzoxépine-5-ol de Formule I et éventuellement à les isoler sous la forme de leurs sels d'addition avec un acide,

f) si on le souhaite, à dédoubler les composés racémiques de Formule I en leurs inverses optiques,

g) si on le souhaite, à préparer les composés N-alkyle par une alkylation ultérieure.

9. Procédé selon la revendication 8, caractérisé en ce qu'il consiste à effecteur l'hydrogénation de la double liaison 3,4 et la réduction du groupe 5-céto selon l'étape a) dans un domaine de pH neutre ou faiblement acide avec du borohydrure de sodium.

10. Procédé selon la revendication 9, caractérisé en ce qu'il consiste à effectuer la réduction dans un domaine de pH acide avec du cyanoborohydrure de sodium.

11. Procédé selon la revendication 9, caractérisé en ce qu'il consiste à effectuer l'hydrogénation avec de l'hydrogène en présence de nickel Raney dans un solvant protique de préférence l'éthanol ou l'isopropanol.

12. Procédé selon la revendication 8, caractérisé en ce qu'il consiste à effectuer l'hydrogénation de la double liaison 3,4 selon l'étape b) dans un domaine faiblement acide au moyen de cyanoborohydrure de sodium.

13. Procédé selon la revendication 12, caractérisé en ce que l'on effectue l'hydrogénation avec de l'hydrogène en présence de nickel Raney dans un solvant aprotique, de préférence le toluène ou le benzène.

14. Procédé selon la revendication 8, caractérisé en ce qu'il consiste à effectuer la réduction du groupe 5-céto en le groupe 5-hydroxy dans un domaine de pH neutre avec du borohydrure de sodium, du borohydrure de lithium, de l'hydrure de lithium et d'aluminium, de l'hydrure de bis-(2-méthoxy-éthoxy)-aluminium et de sodium ou du tri-sec.-butylborohydrure de lithium selon l'étape c).

15. Procédé selon la revendication 14, caractérisé en ce qu'il consiste à effectuer la réduction dans un domaine de pH fortement acide avec du cyanoborohydrure de sodium.

16. Procédé selon l'une quelconque des revendications 8 à 10, 12, 14 à 15, caractérisé en ce qu'il consiste à utiliser en tant que solvant des alcools à faible masse moléculaire, du dioxanne, du tétrahydrofuranne, de diméthylformamide, l'éther diméthylique de l'éthylèneglycol ou l'éther diméthylique du diéthylèneglycol, et à ajuster éventuellement le domaine de pH avec de l'acide acétique, de l'acide p-toluènesulfonique, de l'acide benzènesulfonique, de l'acide chlorhydrique ou de l'acide sulfurique.

17. Médicament se composant d'un ou plusieurs composés selon la revendication 1 et de supports et adjuvants courants.

**Revendications pour l'Etat contractant: AT**

1. Procédé pour la préparation de dérivés de 3-amino-1-benzoxépines de Formule I

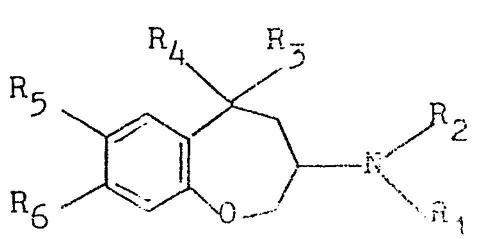

où $R_1$ et $R_2$ sont, indépendamment l'un de l'autre, chacun un atome d'hydrogène, un groupe alkyle en $C_1-C_5$, lequel peut éventuellement porter en position terminale un radical phényle non-substitué ou un radical phényle substitué par un ou deux atomes d'halogène, groupes méthyle ou groupes méthoxy, ou un groupe 3,4-méthylènedioxy ou 3,4-éthylènedioxy, un groupe alkyle en $C_2-C_5$ éventuellement substitué en position terminale par un groupe hydroxy ou méthoxy, ou un groupe alcényle en $C_3-C_4$, ou bien l'un des deux radicaux $R_1$ et $R_2$ est un atome d'hydrogène ou un groupe alkyle en $C_1-C_5$ et l'autre est un groupe alkyle en $C_2-C_5$, substitué en position terminale par un groupe $NR_7R_8$ — dans

lequel $R_7$ et $R_8$ peuvent, indépendamment l'un de l'autre, être chacun un atome d'hydrogène ou un groupe alkyle en $C_1-C_5$, ou où $R_7$ et $R_8$ peuvent être liés l'un à l'autre, directement ou par l'intermédiaire d'un hétéroatome O, S ou N pour donner un noyau pentagonal à heptagonal, ou bien les groupes alkyle $R_1$ et $R_2$ peuvent être liés l'un à l'autre, directement ou par l'intermédiaire d'un hétéroatome O, S ou $NR_9$, pour donner un noyau pentagonal à heptagonal, $R_9$ étant un atome d'hydrogène, un groupe méthyle, benzyle ou phényle, l'un des deux radicaux $R_3$ et $R_4$ étant un atome d'hydrogène et l'autre un groupe hydroxy, ou bien les deux radicaux $R_3$ et $R_4$ sont ensemble l'oxygène, $R_5$ et $R_6$ sont, indépendamment l'un de l'autre, chacun un atome d'hydrogène, un atome d'halogène, un groupe alkyle, alcoxy ou alkylthio, où le radical alkyle peut, chaque fois avoir de 1 à 4 atomes de carbone, ou bien l'un des deux radicaux $R_5$ et $R_6$ est le groupe trifluorométhyle ou nitro et l'autre un atome d'hydrogène, leurs stéréoisomères ainsi que leurs sels d'addition avec un acide, caractérisé en ce qu'il consiste à réduire avec un agent réducteur aux hydrures des dérivés de la 3-amino-1-benzoxépine-5(2H)-one de Formule II

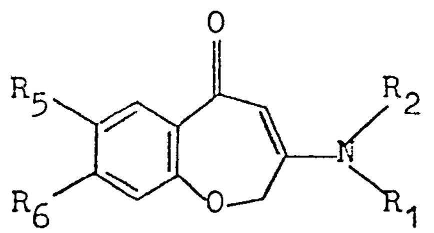

où $R_1$, $R_2$, $R_5$ et $R_6$ ont la signification ci-dessus, dans un solvant inerte dans la plage de températures comprise entre $-70°C$ et la température de reflux du solvant utilisé, et éventuellement en présence d'un acide organique ou inorganique, ou à les soumettre, dans un solvant inerte, à une hydrogénation catalytique avec de l'hydrogène,

a) si on le souhaite, à hydrogéner la double liaison 3,4 et à réduire le groupe 5-céto en un groupe 5-hydroxy et à isoler les dérivés du 2,3,4,5-tétrahydro-3-amino-1-benzoxépine-5-ol de Formule I,
b) si on le souhaite, à hydrogéner la double liaison 3,4 et à isoler les dérivés de la 3-amino-3,4-dihydro-1-benzoxépine-5(2H)-one de Formule I,
c) si on le souhaite, à réduire les dérivés de la 3-amino-3,4-dihydro-1-benzoxépine-5(2H)-one de Formule I en des dérivés de la 2,3,4,5-tétrahydro-3-amino-1-benzoxépine-5-ol de Formule I et à les isoler,
d) si on le souhaite, à convertir les bases libres obtenues en a) à c) en les sels d'addition avec un acide correspondant, ou à transformer les sels d'addition avec un acide en les bases libres,
e) si on le souhaite, à séparer les composés de Formule I obtenus en a) ou c) en les dérivés du rac.-cis- et rac.-trans-2,3,4,5-tétrahydro-3-amino-1-benzoxépine-5-ol de Formule I, et, éventuellement, à isoler ces derniers sous la forme de leurs sels d'addition avec un acide,
f) si on le souhaite, à dédoubler les composés racémiques de Formule I en les inverses optiques,
g) si on le souhaite, à préparer les composés N-alkyle par une alkylation ultérieure.

2. Procédé selon la revendication 1, caractérisé en ce qu'il consiste à effectuer l'hydrogénation de la double liaison 3,4 selon l'étape b) dans un domaine faiblement acide, au moyen de cyanoborohydrure de sodium.

3. Procédé selon la revendication 1, caractérisé en ce qu'il consiste à effectuer la réduction du groupe 5-céto en le groupe 5-hydroxy dans une zone de pH neutre avec du borohydrure de sodium, du cyanoborohydrure de lithium, de l'hydrure de lithium et d'aluminium, de l'hydrure de bis(2-méthoxy-éthoxy) aluminium et de sodium ou du tri-sec.-butylborohydrure de lithium selon l'étape c).

4. Procédé selon la revendication 1, caractérisé en ce qu'il consiste à préparer, en tant que composés de Formule I, des rac.-cis- ou rac.-trans-2,3,4,5-tétrahydro-3-A-1-benzoxépine-5-ols où A signifie les radicaux amino, méthylamino, diméthylamino, diéthylamino, $\beta$-méthoxyéthylamino, isopropylamino, n-butylamino, benzylamino, phénéthylamino, morpholino, pyrrolidino, pipéridino, $\gamma$-diméthylamino-propylamino ou $\beta,\beta$-diméthyl-$\gamma$-diméthylamino-propylamino.

5. Procédé selon la revendication 1, caractérisé en ce qu'il consiste à préparer en tant que composés de Formule I des rac.-cis- ou rac.-trans-2,3,4,5-tétrahydro-3-méthylamino-7B,8C-1-benzoxépine-5-ols où B est l'hydrogène, le chlore, le brome, le méthyle, l'éthyle ou le méthoxy, et C est l'hydrogène, le chlore, le méthyle ou le méthoxy.

6. Procédé selon la revendication 1, caractérisé en ce qu'il consiste à préparer en tant que composés de Formule I des rac.-3-A-3,4-dihydro-1-benzoxépine-5(2H)-ones où A signifie le radical amino, méthylamino, diméthylamino, isopropylamino, n-butylamino, benzylamino, phénéthylamino, morpholino, pyrrolidino, pipéridino, $\beta$-méthoxyéthylamino, $\beta$-diméthylamino-éthylamino ou $\gamma$-diméthylamino-

propylamino.

7. Procédé selon la revendication 1, caractérisé en ce qu'il consiste à préparer en tant que composés de Formule I des rac.-3-méthylamino-3,4-dihydro-7B,8C-1-benzoxépine-5(2H)-ones où B est l'hydrogène, le chlore, le brome, le méthyle, l'éthyle ou le méthoxy et C est l'hydrogène, le chlore, le méthyle ou le méthoxy.